Europäisches Patentamt

European Patent Office

Office européen des brevets

(19)

(11) Publication number: **0 136 090**
**A2**

# EUROPEAN PATENT APPLICATION

(12)

(21) Application number: **84305839.7**

(22) Date of filing: **24.08.84**

(51) Int. Cl.⁴: **C 12 N 15/00, C 07 H 21/04, C 12 N 1/20 // (C12N1/20, C12R1:19)**

(30) Priority: **26.08.83 US 526547**
**05.07.84 US 628145**

(43) Date of publication of application: **03.04.85**
**Bulletin 85/14**

(84) Designated Contracting States: **CH DE FR GB LI**

(71) Applicant: **E.I. DU PONT DE NEMOURS AND COMPANY, 1007 Market Street, Wilmington Delaware 19898 (US)**

(72) Inventor: **Arentzen, Rene, 2302 Larkwood Drive, Wilmington Delaware 19810 (US)**
Inventor: **Petteway, Stephen Robert, Jr., 42 Winding Hill Drive Mendenhall, Hockessin Delaware 19707 (US)**

(74) Representative: **Hildyard, Edward Martin et al, Frank B. Dehn & Co. European Patent Attorneys Imperial House 15-19 Kingsway, London WC2B 6UZ (GB)**

(54) **Improvements in or relating to expression plasmids.**

(57) A promoter-operator Shine-Dalgarno ClaI-containing fragment has been obtained comprising a —10 region and a Shine-Dalgarno sequence characteristic of the trp promoter-operator Shine-Dalgarno fragment and comprising in addition the following sequences:

(i) a 6-base pair sequence in the —35 region of the fragment (5'→3') of

$$\begin{array}{c} T \; G \; C \; C \; G \; A \\ A \; C \; G \; G \; C \; T \end{array} , \quad \text{and}$$

(ii) a sequence of about 17 base pairs between the —35 and the —10 regions of said fragment, the sequence of the first eight base pairs (5'→3') being

$$\begin{array}{c} C \; G \; C \; G \; C \; A \; T \; C \\ G \; C \; G \; C \; G \; T \; A \; G \end{array} .$$

Plasmids have been prepared comprising this promotor-operator fragment and plasmids have also been prepared comprising the trp promoter-operator, which are useful for the over-production in bacteria of particular proteins, such as β-lactamase, β-interferon and interleukin 2.

ACTORUM AG

0136090

1

"Improvements in or relating to expression
plasmids"

This invention concerns inducible expression
plasmids for overproducing proteins such as
interleukin-2, ß-interferon and ß-lactamase in
microorganisms, e.g. E. coli.

Developments in recombinant DNA technology
have made it possible to isolate specific genes or
portions thereof from higher organisms, such as man
and other mammals, and to transfer the genes or
fragments to a microorganism species, such as
bacteria or yeast.  The transferred gene is
replicated and propagated as the transformed
microorganism replicates.  As a result, the
transformed microorganism may have the capacity to
make whatever protein the gene or fragment encodes,
whether it be an enzyme, a hormone, an antigen, an
antibody, or any other protein.

This technology has already been employed to
modify bacteria for the production of such useful
polypeptide products as somatostatin (Itakura et al.,
Science 198, page 1056 to 1063 (1977)); the component
A and B chains of human insulin (Goeddel et al.,
Proc. Natl. Acad. Sci. USA, 76, page 106 to 110
(1979)); human growth hormone (Goeddel et al., Nature
281, page 544 to 548 (1979)); and human interferon (GB 2,068,970 A). U.S. Patent 4,322,499 in Columns 2 to 7 summarizes several of the techniques that have been employed in recombinant DNA technology.

Human interleukin-2 (IL-2), a glycoprotein having a molecular weight of about 15,000 daltons, has also been produced by recombinant DNA technology. Taniguchi et al., in Nature 302, at pages 305 to 310 (1983) disclose the cloning of a cDNA coding for IL-2 and its expression in cultured monkey cells to produce a biologically active peptide. Fujita et al., in Proc. Natl. Acad. Sci. USA 80, at pages 7437 to 7441 (1983) disclose the sequence of the IL-2 gene. Devos et al., in Nucleic Acids Research 11, at pages 4307 to 4323 (1983) and Rosenberg et al., in Science 223, at pages 1412 to 1415 (1984) disclose expression of the IL-2 gene in E. coli under control of the trp promoter to obtain a biologically active IL-2 product at a concentration of about 5 to 10% of the total protein.

Many recombinant DNA techniques employ two classes of compounds, transfer vectors and restriction enzymes. A transfer vector is a DNA molecule which contains, inter alia, genetic information to insure its own replication when transferred to a host microorganism strain. Examples of transfer vectors commonly used in bacterial genetics are plasmids and the DNA of certain bacteriophages. "Plasmid" is the term applied to any autonomously replicating DNA unit which might be found in a microbial cell, other than the genome of the host cell itself.

"Restriction enzyme" is the term applied to hydrolytic enzymes capable of catalyzing the cleavage of DNA molecules. Many of these enzymes recognize specific short sequences in DNA and will cleave or catalyze the cleavage of DNA into precise, moderately short fragments depending upon the particular enzyme and the sequence of DNA which it recognizes. After cleavage, heterologous genes or gene fragments can be inserted into the plasmid by endwise joining at the cleavage site or at reconstructed ends adjacent the cleavage site. The term "heterologous" refers to a gene not ordinarily found in a particular microorganism such as E. coli, or a polypeptide sequence ordinarily not produced by that microorganism. The term "homologous" refers to a gene or polypeptide sequence ordinarily found in or produced by a particular microorganism.

When the gene is properly inserted with reference to portions of the plasmid which govern the transcription and translation of the encoded DNA message, the resulting vehicle can be used to express the protein for which the inserted gene codes. The term "expression" in recombinant DNA technology refers to the operation by which the protein coded by the transferred gene is actually synthesized by the transformed organism.

The manner by which gene expression is controlled in E. coli is well understood. Genes which code for proteins that carry out related functions in the cell tend to cluster in continuous sequence. The cluster is called an "operon". All genes in the operon are transcribed in the same direction, beginning with the codons coding for the N-terminal amino acid of the first protein in the sequence and continuing through to the C-terminal end of the last protein in the operon.

At the beginning of the operon, proximal to the N-terminal amino acid codon, there exists a region of the DNA, termed the control region, which

4

includes a variety of controlling elements including the promoter-operator and sequences for the ribosomal binding site (Shine-Dalgarno sequence). The function of these sites is to permit the expression of those genes under their control to be responsive to the needs of the organism. The control region functions that must be present for expression to occur are transcription into a messenger RNA complementary to the DNA template and the translation of the messenger RNA into protein on the cellular organelles called ribosomes.

Expression of the first gene in the sequence is initiated by transcription at the position coding for the N-terminal amino acid of the first protein of the operon. The expression of each gene downstream from that point is also initiated in turn, at least until a termination signal or another operon is encountered with its own control region, keyed to respond to a different set of environmental cues. While there are many variations in detail on this general scheme, the important fact is that, to be expressed in a prokaryote such as E. coli, a gene must be properly located with respect to a control region having transcription and translation functions. The slight alteration of a promoter sequence can increase the probability that RNA polymerase will bind to the promoter, and so enhance the rate of transcription. Mutations in operator regions or in a regulator gene can prevent the binding of a repressor and thereby greatly increase transcription.

If recombinant DNA technology is to fully sustain its promise, systems must be devised which optimize expression of gene inserts, so that the intended proteins can be made available in high

yield. Commonly used ß-lactamase and lactose promoter-operator systems have not fully utilized the capacity of the technology from the standpoint of yield. A more efficient promoter-operator system, the tryptophan (trp) promoter-operator, has been employed to enhance expression of foreign genes in bacteria. For instance, European Patent Application 36,776 describes plasmid expression of human growth hormone employing a trp promoter-operator system from which the attenuator region has been deleted.

Hallewell et al., Gene 9, page 27 to 47 (1980), describe derivatives of plasmid pBR322 suitable for expression of foreign genes which contain the E. coli trp promoter, the trpE gene, and about 15% of the trpD gene. Edman et al., Nature 291, page 503 to 506 (1981), describe the construction of recombinant plasmids which direct the synthesis of hepatitis B core antigen and a ß-lactamase/hepatitis B surface antigen fusion polypeptide under the control of the trp operon regulating region.

Russell et al., Gene 17, page 9 to 18 (1982), describe the construction of several novel trp-promoter-operator-bearing plasmids by cloning a 41-bp AluI restriction fragment from the trp promoter-operator region into the PvuII site of pBR322, regenerating PvuII sites. Christie et al., J. Mol. Biol. 143, pages 335 to 341 (1980) disclose the plasmid pLD102. Guarente et al., Cell 20, page 543 to 553 (1980) disclose the plasmid pLG400.

European Patent Application 52,002 discloses a plasmid having an insertion site for a DNA fragment adjacent to a trp promoter, the attenuator of which has been removed. European Patent Application 48,970 discloses the preparation of mature human fibroblast interferon using a plasmid which contains the trp promoter system. European Patent Application 67,540 discloses a hybrid promoter-operator which contains a promoter-operator fragment capable of being chemically induced, e.g., lac, and a fragment of a second promoter having a greater signal strength, e.g., trp. U.K. Patent Application 2,104,901A discloses the use of a portable promoter, e.g., lac and trp, for expression of foreign genes in E. coli. The use of a lac operon is exemplified.

## SUMMARY OF THE INVENTION

This invention concerns novel inducible plasmids useful for the overproduction in bacteria of a number of homologous and heterologous proteins. A representative bacterium in which the plasmids can be employed is E. coli and representative proteins that can be overproduced include interleukin-2, ß-interferon (IFN-ß) and ß-lactamase. This invention also concerns a novel promoter-operator fragment, microorganisms transformed by the plasmids of this invention, a method for making the plasmids, a method for incorporating them into microorganisms, and a method for overproducing the encoded protein(s). By "biologically pure culture", used to describe microorganisms of this invention, is meant a genetically homogeneous culture.

The novel promoter-operator fragment of this invention is characterized by having

    (i)   a 6-base pair sequence in the -35 region of the fragment (5' $\longrightarrow$ 3') of

$$\underline{\text{T}}\ \underline{\text{G}}\ \underline{\text{C}}\ \underline{\text{C}}\ \underline{\text{G}}\ \underline{\text{A}}$$
$$\text{A}\ \text{C}\ \text{G}\ \text{G}\ \text{C}\ \text{T}$$
, and

    (ii)  a sequence of about 17 base pairs between the -35 and the -10 regions of said fragment, the sequence of the first eight base pairs (5' $\longrightarrow$ 3') being

7

$$\underline{C} \ \underline{G} \ \underline{C} \ \underline{G} \ \underline{C} \ A \ T \ \underline{C}$$
$$G \ C \ G \ C \ G \ T \ A \ G$$

.

The plasmids of this invention include those that comprise the promoter-operator fragment described herein having sequences (i) and (ii). They also include pKGP43, pBREIL-2, pKGP13-19R•trp6, pKGP36•trp and pTrpEIL-2. Representative plasmids of this invention are the following:

pKGP36, see Figure 4A

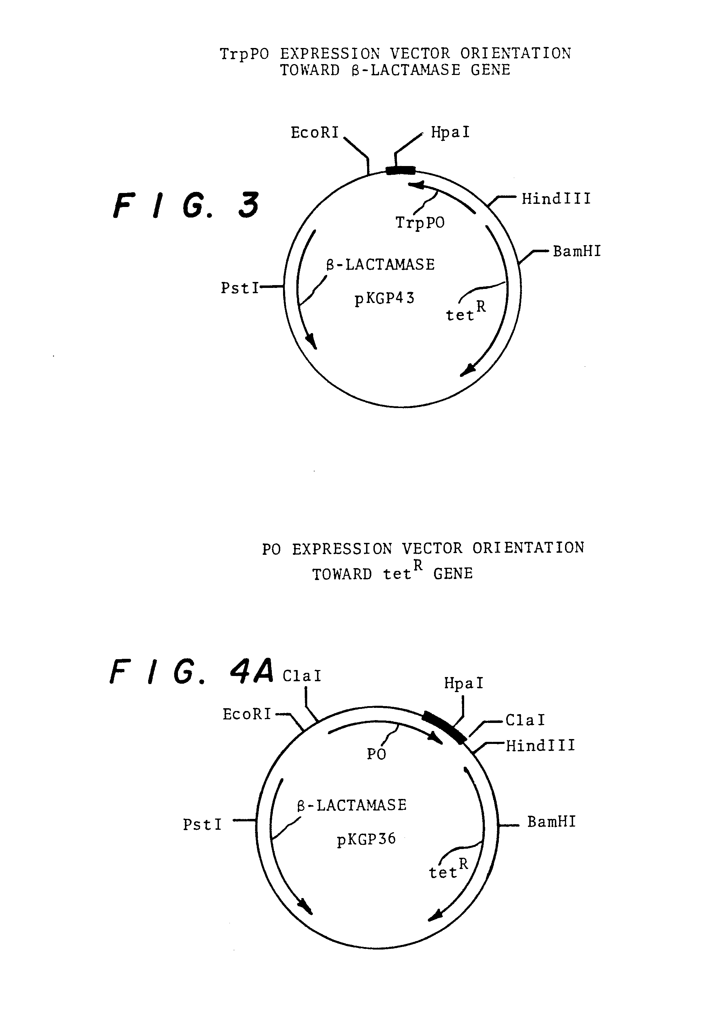

pKGP43, Figure 3

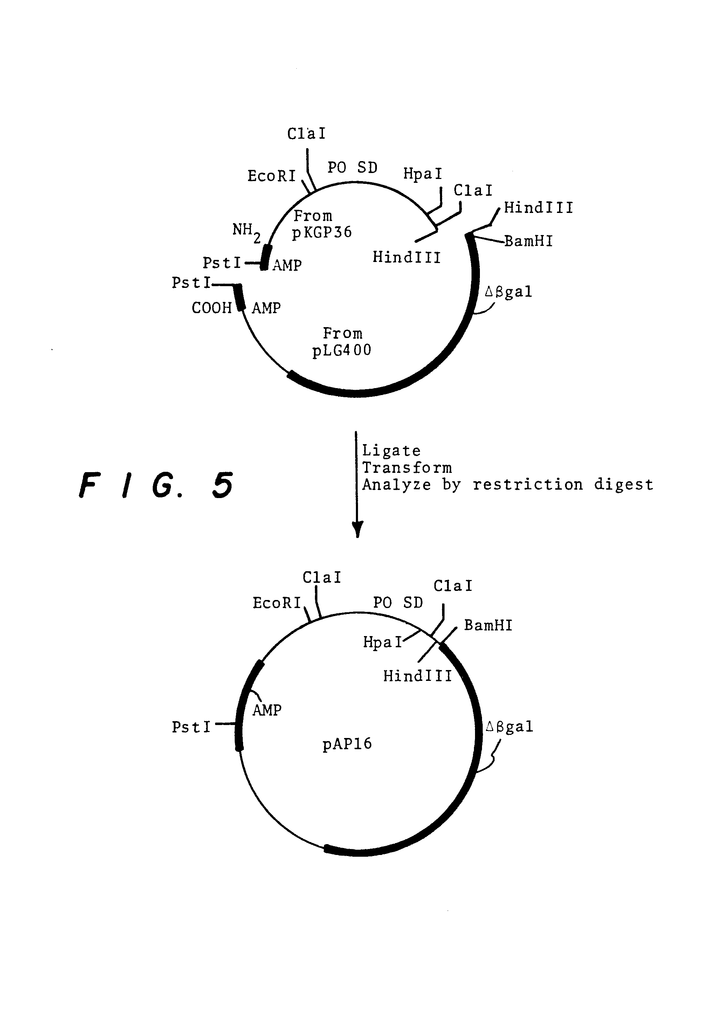

pAP16, Figure 5

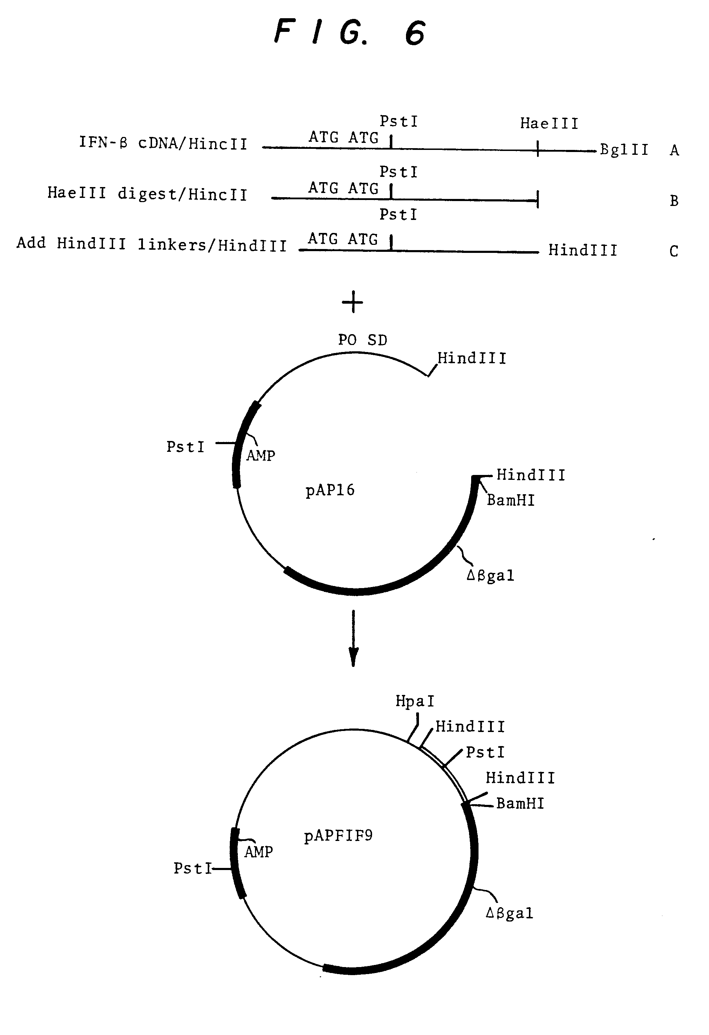

pAPFIF9, Figure 6

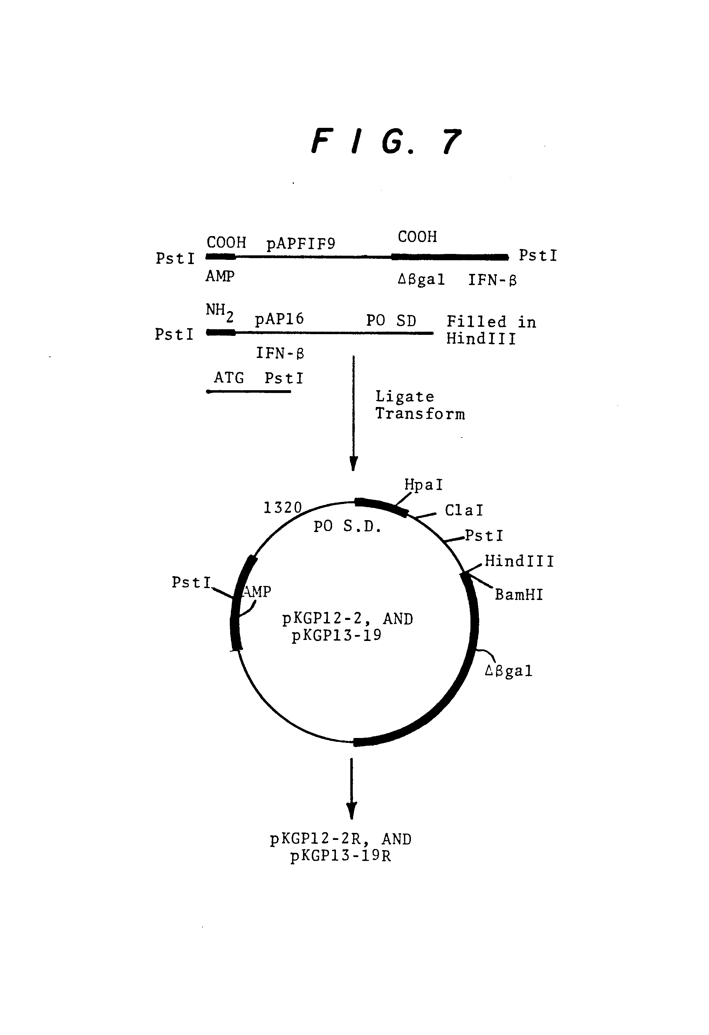

pKGP12-2, Figure 7

pKGP13-19, Figure 7

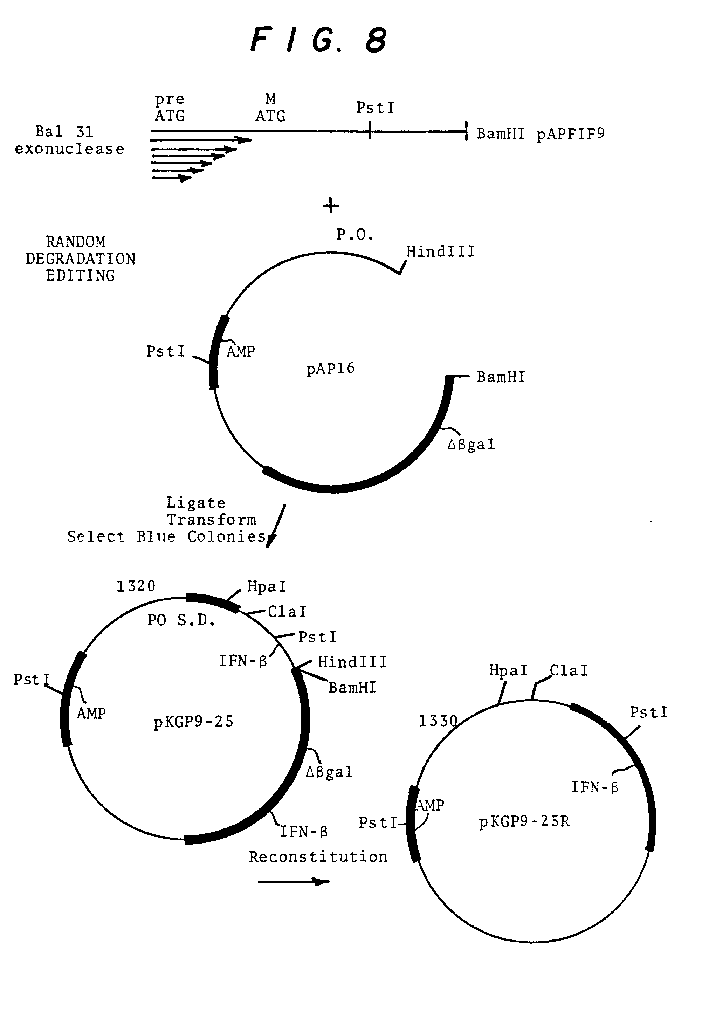

pKGP9-25, Figure 8

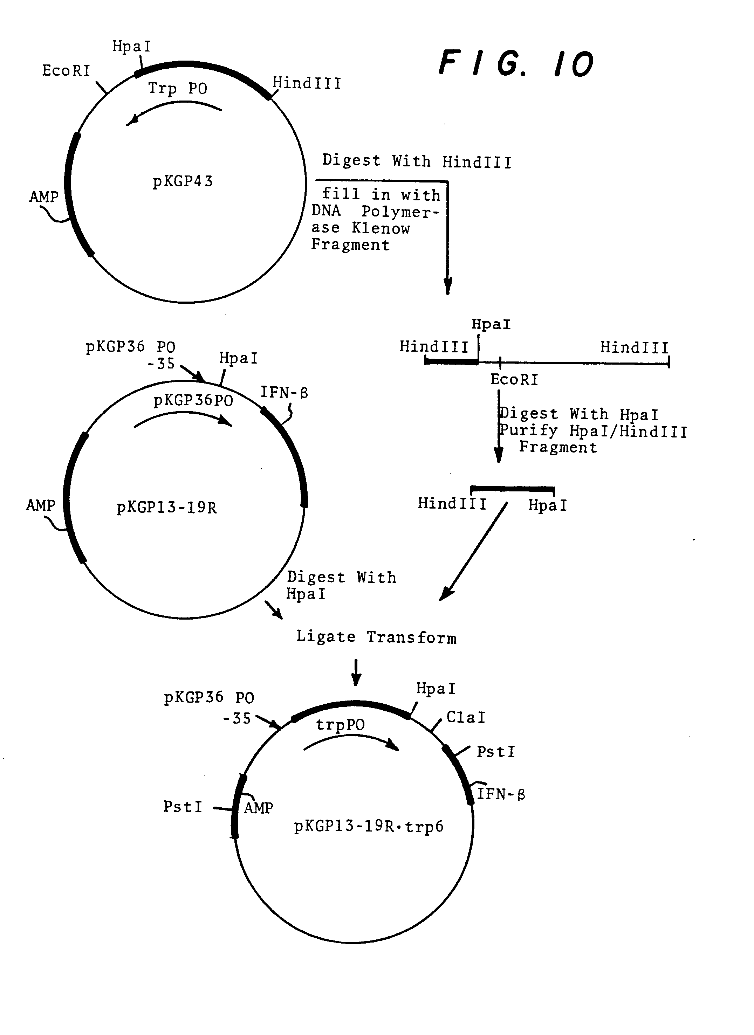

pKGP13-19R•trp6, Figure 10

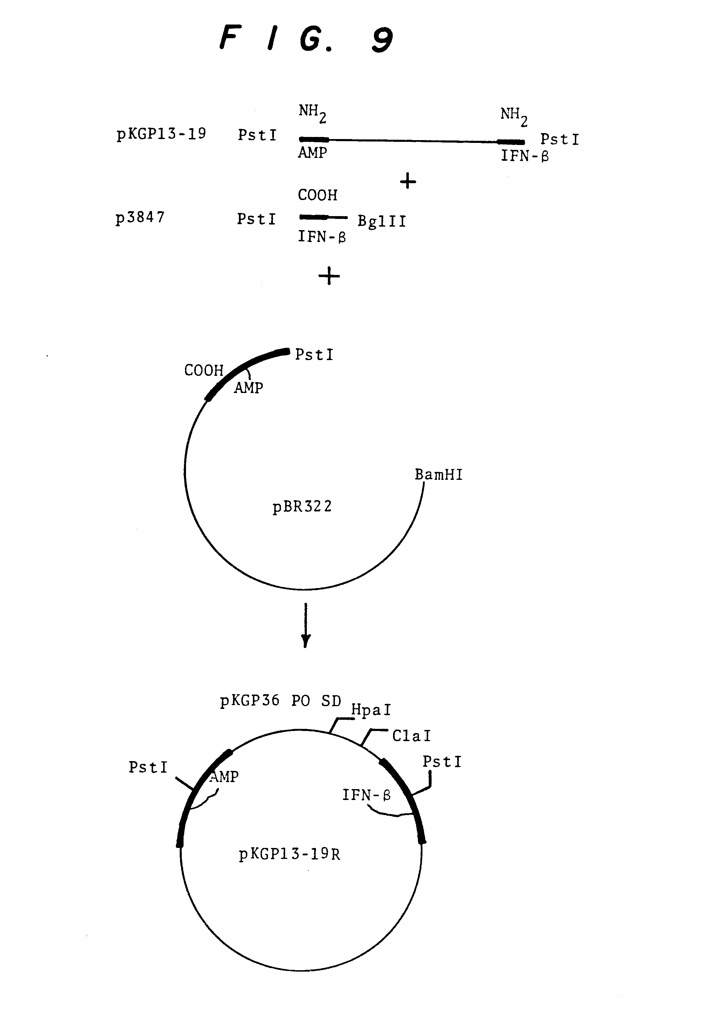

pKGP13-19R, Figure 9

pKGP9-25R, Figure 8

pKGP12-2R, Figure 7

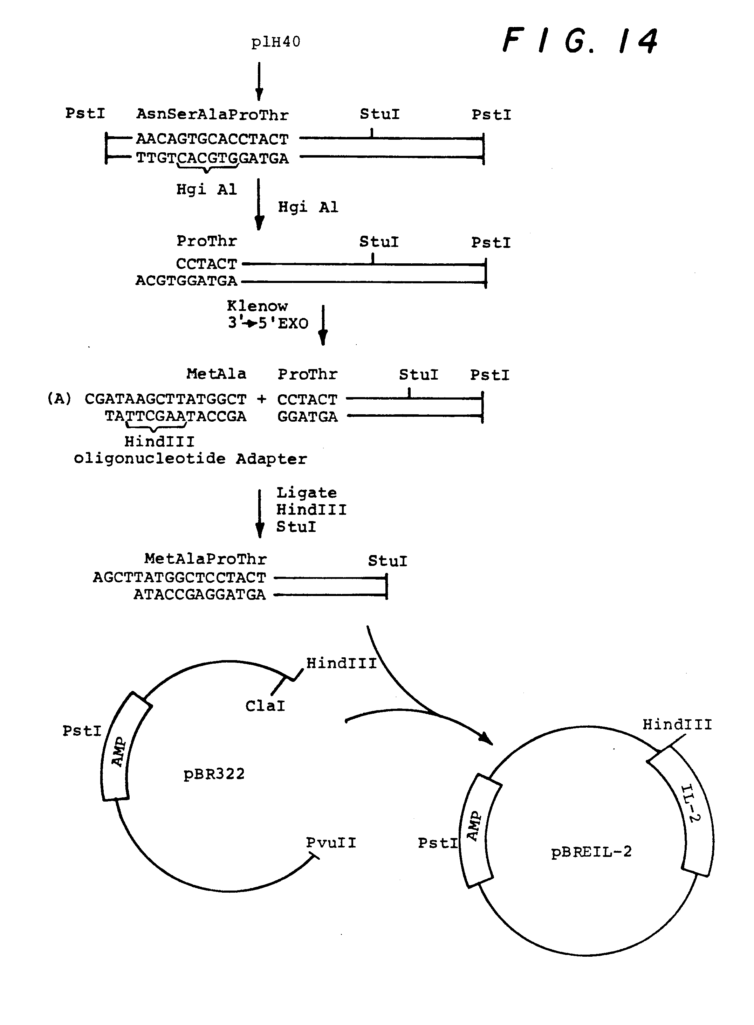

pBREIL-2, Figure 14

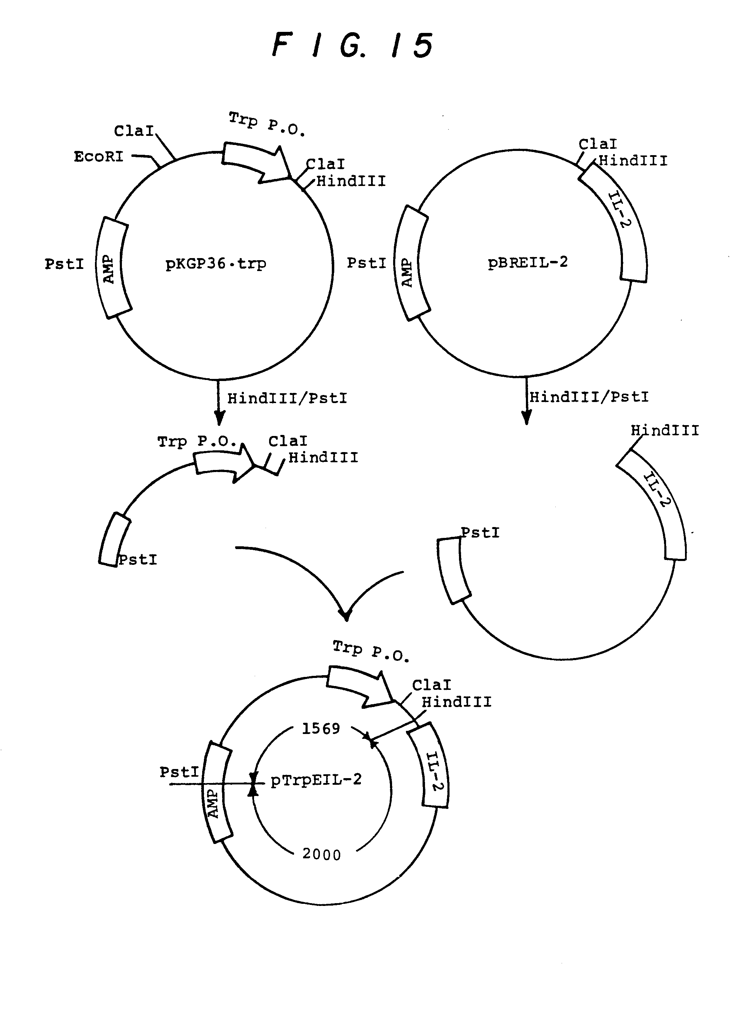

pKGP36•trp, Figure 15

pTrpEIL-2, Figure 15.

This invention also encompasses said plasmids that have mutated somewhat with the passage of time.

The method for making plasmids pKGP36 and pKGP43 comprises recombining fragments of known plasmids of the pLD102 and pBR322 types in the following manner:

(i) restricting a plasmid of the pLD102 type using HpaII and BalI;

(ii) isolating and purifying the trp-promoter-operator-containing HpaII/BalI fragment;

(iii) modifying the HpaII/BalI fragment by limited digestion with TaqI; and

0136090

8

(iv)   inserting said modified fragment into the
       ClaI site of pBR322.

From the reaction mixture was separated trp
P.O.-containing pKGP43 as well as pKGP36 which
comprises the novel promoter-operator fragment of
this invention.

The method for making plasmid pAP16 of the
invention comprises the steps of:

(i)    restricting a plasmid of the pLG400 type
       using PstI and HindIII to obtain a
       PstI/HindIII fragment;

(ii)   restricting a plasmid of the pKGP36 type
       with PstI and HindIII, and isolating and
       purifying the promoter-operator-containing
       fragment; and

(iii)  ligating the promoter-operator containing
       fragment of pKGP36 to the PstI/HindIII
       fragment of pLG400.

The method for making plasmid pAPFIF9 of the
invention comprises the steps of:

(i)    restricting the IFN-ß cDNA fragment with
       HaeIII/HincII to obtain a HaeIII/HincII
       fragment, and isolating and purifying said
       fragment;

(ii)   ligating HindIII linkers to both ends of the
       HaeIII/HincII fragment of step (i) to obtain
       a HindIII/HindIII fragment, and isolating
       and purifying said fragment;

(iii)  restricting a plasmid of the pAP16 type
       using HindIII; and

(iv)   ligating the fragment of step (ii) with the
       fragment of step (iii).

The method for making plasmids pKGP12-2 and
pKGP13-19 of the invention comprises the steps of:

9

(i)      restricting a plasmid of the pAP16 type
         using HindIII, and filling in the HindIII
         site with the Klenow fragment of E. coli DNA
         polymerase to obtain a blunt-ended fragment;

(ii)     restricting the fragment of step (i) using
         PstI to obtain a PstI fragment containing
         the amino terminal of the ß-lactamase (AMP)
         gene and the pKGP36 tryptophan
         promoter-operator Shine-Dalgarno (P.O.-S.D.)
         unit, and purifying said fragment;

(iii)    restricting a plasmid of the pAPF1F9 type
         using PstI, and purifying the fragment which
         contains the carboxyl terminal of the
         ß-lactamase gene and the carboxyl terminal
         of IFN-ß cDNA;

(iv)     editing the IFN-ß cDNA fragment giving a 140
         base pair blunt ended ATG/PstI fragment; and

(v)      ligating the fragments of steps (ii), (iii)
         and (iv).

         The method for making plasmid pKGP9-25 of
the invention comprises the steps of:

(i)      restricting a plasmid of the pAP16 type
         using HindIII, and filling in the HindIII
         site with the Klenow fragment of E. coli DNA
         polymerase to obtain a blunt-ended fragment;

(ii)     digesting the fragment of step (i) with
         BamHI;

(iii)    restricting a plasmid of the pAPF1F9 type
         with HpaI to give three fragments;

(iv)     treating the fragments of step (iii) with
         Bal 31 exonuclease and BamHI to give
         fragments randomly degraded at the 5' end of
         the IFN-ß gene and ending in an BamHI site;

(v)      ligating the IFN-ß fragments of step (iv)
         into the pAP16 fragment of step (ii).

The method for making plasmids pKGP12-2R, pKGP13-19R, and pKGP9-25R of the invention comprises the steps of:

(i) restricting a plasmid of the pBR322 type using PstI and BamHI to obtain a PstI/BamHI fragment, and purifying said fragment;

(ii) restricting a plasmid of the (a) pKGP12-2, (b) pKGP13-19, or (c) pKGP9-25 type using PstI, and purifying the fragment which contains the amino terminal of ß-lactamase, the pKGP36 P.O.-S.D. unit and the edited amino terminal of the IFN-ß gene;

(iii) restricting a plasmid of the p3847 type using PstI and BglII and purifying the fragment which contains the carboxyl terminal fragment of the IFN-ß gene; and

(iv) ligating the fragments of steps (i), (iii), and (a), (b) or (c) of step (ii).

The method for making plasmid pKGP13-19R•trp6 of the invention comprises the steps of:

(i) restricting a plasmid of the pKGP43 type using HindIII and filling in the HindIII sites with the Klenow fragment of E. coli DNA polymerase to obtain a blunt-ended fragment;

(ii) restricting the fragment of step (i) using HpaI and isolating the HpaI/ filled in HindIII fragment containing the -35 region of the trp promoter;

(iii) restricting a plasmid of the pKGP13-19R type using HpaI; and

(iv) ligating the fragment of step (ii) with the fragment of step (iii)

The method for making plasmid pBREIL-2 of the invention comprises the steps of:

(i) restricting a plasmid of the plH40 type with PstI to produce a cDNA fragment containing the gene for IL-2;

(ii)   treating the fragment of step (i) to form a
       blunt 3' end beginning with the CCT codon;

(iii)  ligating to the blunt 3' end of the fragment
       of step (ii) an adapter molecule comprising
       ATG and GCT codons in serial arrangement and
       containing a HindIII/site;

(iv)   restricting the fragment of step (iii) with
       HindIII and StuI;

(v)    restricting a plasmid of the pBR322 type
       with HindIII and PvuII, and isolating the
       large DNA fragment;

(vi)   ligating the IL-2 gene-containing fragment
       of step (iv) to the HindIII/PvuII fragment
       of step (v).

The method for making plasmid pKGP36•trp of
the invention comprises the steps of:

(i)    restricting a plasmid of the pKGP36 type
       with HpaI and PstI and purifying the
       fragment containing the $tet^R$ gene;

(ii)   restricting a plasmid of the pKGP13-19R•trp-
       6 type with HpaI and PstI, and purifying the
       fragment containing the trp P.O.;

(iii)  ligating the fragments of steps (i) and (ii).

The method for making plasmid pTrpEIL-2 of
the invention comprises the steps of:

(i)    restricting a plasmid of the pBREIL-2 type
       with HindIII and PstI, and purifying the
       fragment containing the IL-2 gene;

(ii)   restricting a plasmid of the pKGP36•trp type
       with HindIII and PstI, and purifying the
       fragment containing the trp P.O.;

(iii)  ligating the fragments of steps (i) and (ii).

The method for modifying microorganisms to
include the novel plasmids of this invention
comprises the steps of:

12

(i)    growing bacterial cells to a designated optical density;

(ii)   modifying the cell walls by treating with calcium chloride solution;

(iii) mixing competent cells with ligated DNA mixture containing the novel plasmid; and

(iv)  incubating the mixture to effect insertion of the plasmid.

Analyzing for the modified microorganisms that now contain the cloned plasmid is accomplished by plating the incubated cells of step (iv) on selected media and then assaying.

The method for overproducing proteins from the modified bacterial cells of this invention comprises the steps of:

(i)    growing the modified bacterial cells in an enriched medium to an optical density of at least 2 at 600 nm;

(ii)   diluting the grown cells 1:10 in minimal media; and

(iii) treating the diluted cells with ß-indoleacrylic acid (ß-IAA).

The medium comprising the diluted cells and ß-IAA can be analyzed for overproduced protein by assaying it for activity associated with the protein of interest, such as interleukin-2 and/or ß-lactamase and/or IFN-ß activity. Alternatively, the proteins produced can be labeled with a radioactive amino acid, and the radiolabeled proteins separated and analyzed.

BRIEF DESCRIPTION OF THE DRAWINGS

The plasmids depicted in the Figures are not drawn to scale; base pair distances have been measured clockwise.

Figure 1 depicts the prior art plasmid pLD102 comprised of the vector pACYC177 and the

tryptophan deletion trp Δ LD102 sequence that fuses
the C gene of the tryptophan operon to the tryptophan
leader ribosome binding site.

Figure 2 depicts the prior art plasmid
pBR322.

Figure 3 depicts the
restriction-endonuclease map of novel plasmid pKGP43,
comprising the ß-lactamase gene in which the P.O.
orientation is in the direction of the ß-lactamase
gene.

Figure 4A depicts the
restriction-endonuclease map of novel plasmid pKGP36,
comprising the ß-lactamase gene in which the P.O. is
oriented in the direction of the tetracycline
resistance (tet$^R$) gene.

Figure 4B depicts a comparison of the novel
P.O. fragment from pKGP36 vs. the trp P.O. fragment.

Figure 5 depicts the restriction-endonuclease
map of novel plasmid pAP16 comprising the P.O. from
pKGP36 fused to the ß-galactosidase gene.

Figure 6 depicts novel plasmid pAPFIF9 and
the method for its preparation hereinbefore described.

Figure 7 depicts the restriction-
endonuclease maps of the novel plasmids pKGP12-2 and
pKGP13-19, and their preparation using a three-way
ligation of the pAP16 PstI blunt end fragment, the
pAPFIF9 PstI fragment, and the edited PstI blunt end
IFN-ß fragment.

Figure 8 depicts an alternative editing
method for preparation of plasmid pKGP9-25 employing
pAPFIF9 and Bal 31 exonuclease and ligation into
pAP16.

Figure 9 depicts a three-way ligation of the
PstI/ BamHI fragment from pBR322, the PstI fragment from pKGP13-19, and the PstI/BglII carboxyl terminal IFN-ß fragment to prepare novel plasmid pKGP13-19R.

Figure 10 depicts digestion, ligation and transformation steps leading to formation of novel plasmid pKGP13-19R•trp6.

Figure 11 depicts the cDNA sequence of the IFN-ß gene from plasmid p3847.

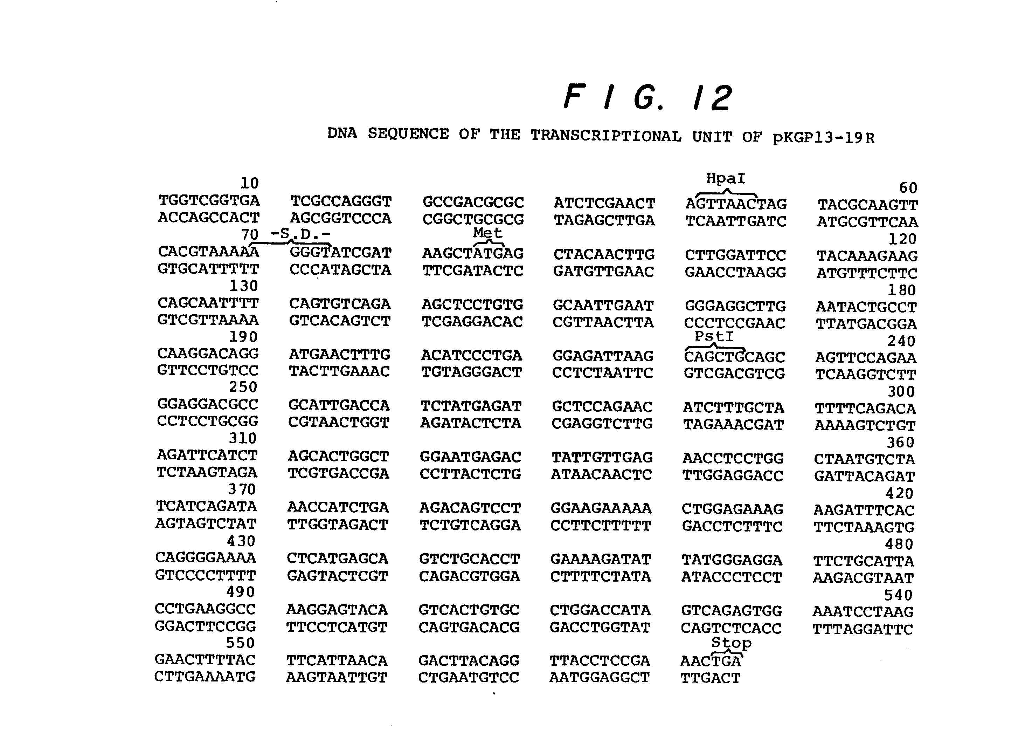

Figure 12 depicts the DNA sequence of the transcriptional unit comprising the P.O.-S.D. fragment that characterizes plasmids pKGP12-2R, pKGP13-19R and pKGP9-25R of this invention.

Figure 13 depicts an approximately 400 bp fragment containing the HpaI site from pLD102.

Figure 14 depicts preparation of the pBREIL-2 vector by ligating the edited IL-2 gene fragment from p1H40 to the larger HindIII/PvuII fragment obtained by restriction of pBR322.

Figure 15 depicts pKGP36•trp and pTrpEIL-2 and preparation of the latter by ligating the PstI/HindIII trp P.O. fragment from pKGP36•trp to the fragment from pBREIL-2 comprising the IL-2 gene and part of the ampicillin gene.

## DETAILS OF THE INVENTION

While the expression of foreign genes in E. coli is generally enhanced by the presence of efficient promoters such as the tryptophan promoter, plasmids expressing genes under control of this promoter may be unstable. Cloning, editing, and manipulation of foreign genes can be difficult due to deletions and rearrangements. One characteristic of this invention concerns provision of an "inefficient" promoter-operator, one that can be used for cloning and editing foreign genes with great stability and which can then be converted to an efficient promoter after gene manipulation is complete. An advantage of

0136090

15

such a system is the long-term maintenance of sequences required for foreign gene expression.

Plasmids And Microorganisms

A number of the novel plasmids of this invention are described with reference to their restriction-endonuclease maps and DNA sequences set out in the Figures. One of the most characteristic regions of certain of these plasmids is the fragment comprising the promoter-operator and Shine-Dalgarno sequences emanating from a unique source and containing a sequence unknown in the art.

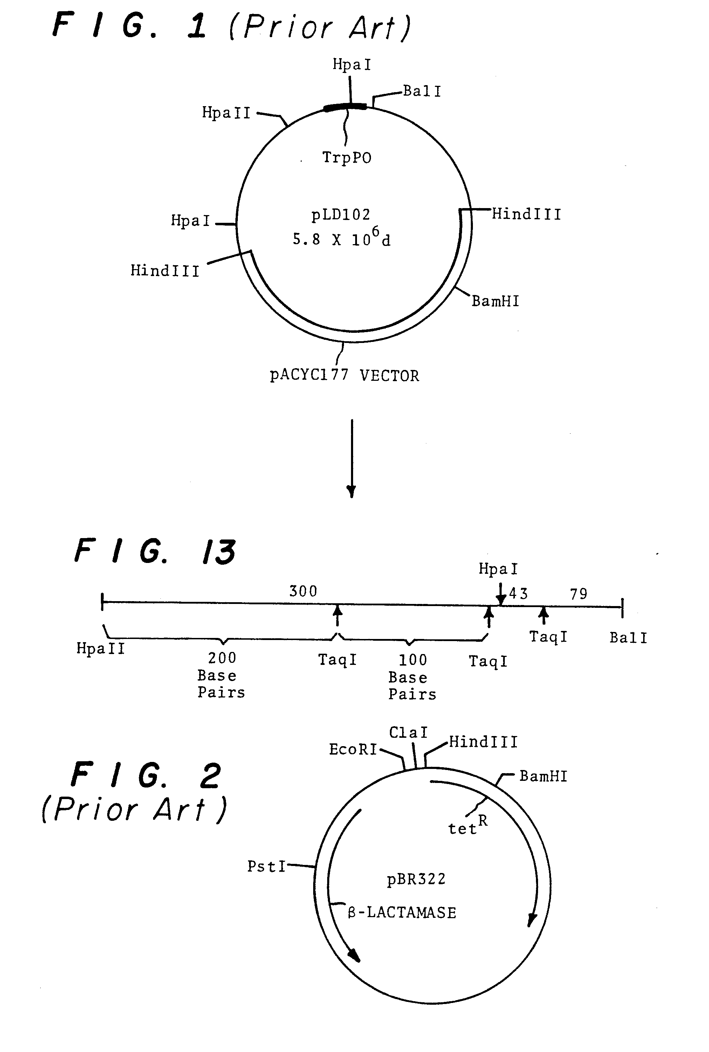

A preferred embodiment of this invention is the plasmid depicted in Figure 3, pKGP43. The HpaI site located between the ClaI and HindIII sites describes the presence and orientation (depicted by arrow) of the P.O.-S.D. fragment. In this novel plasmid, the ß-lactamase gene is oriented downstream from the tryptophan P.O.-S.D. fragment. The Shine-Dalgarno sequence is described by Shine et al., Nature 254, page 34 to 38 (1975). The map is constructed on the basis of the known size and restriction map of pBR322 (Figure 2); the known size and restriction map of the tryptophan P.O.-S.D. fragment from pLD102 (Figure 1); as well as independent restriction mapping and DNA sequence analysis of the composite plasmid pKGP43.

The molecular weight of pKGP43 is 2.8 x $10^6$ daltons (about 4700 bp). The presence of the HpaII/TaqI fragment (tryptophan P.O.-S.D.-containing fragment) confers on the plasmid the ability to code for the overproduction of ß-lactamase in the absence of tryptophan or the presence of ß-IAA or both. The base sequence in the above described P.O.-S.D.-containing fragment does not alter the stability of the composite plasmid.

16

The following distances in base pairs further characterize the plasmid, pKGP43: PstI-EcoRI, 750 bp; EcoRI-HpaI, 100 bp; HpaI-HindIII, 300 bp; HindIII-BamHI, 345 bp. The above distances were determined by agarose and polyacrylamide gel electrophoresis and are approximate. The EcoRI and PstI single restriction sites can be used for the insertion and expression of foreign genes.

Another preferred embodiment of this invention is the plasmid depicted in Figure 4A, pKGP36. The HpaI site locates and orients the P.O.-S.D. sequences. In this novel plasmid, molecular weight of about $3.0 \times 10^6$ daltons, the tet$^R$ gene is downstream from the P.O.-S.D. fragment. The sizes and arrangement of restriction fragments were determined as described for pKGP43. The presence of the P.O.-S.D. fragment confers on the plasmid the ability to express at low levels foreign genes cloned in the HindIII or BamHI restriction sites. After cloning and gene manipulation, the P.O. of pKGP36 can be reconstituted to form the efficient trp P.O. in a simple one-step cloning. The resulting plasmid can then direct the synthesis of high yields of the cloned protein. The following distances in base pairs describe the plasmid; PstI-EcoRI, 750 bp; EcoRI-ClaI, 23 bp; ClaI-HpaI, 565 bp; ClaI-ClaI, 600 bp; HpaI-ClaI, 35 bp; HpaI-HindIII, 39 bp; HindIII-BamHI, 345 bp. The promoter region of pKGP36 was further characterized by sequence analysis.

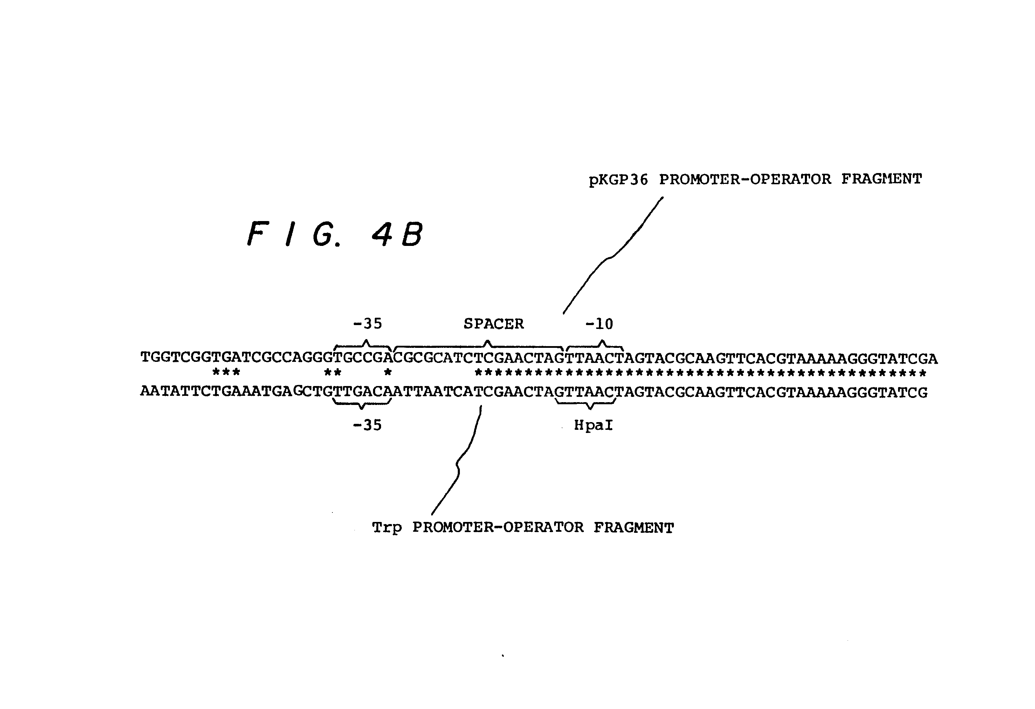

Plasmid pKGP36 contains a novel P.O. sequence oriented toward the tet$^R$ gene of pBR322. The novel P.O. is comprised of the -10 region of the trp P.O. and a novel -35 region that was fused to the trp-10 sequence via a TaqI site during cloning; see Figure 4B. The new sequence contains a novel -35

17

region of six base pairs beginning with a T:A base pair and ending with an A:T base pair (5' $\longrightarrow$ 3'). These two base pairs border the consensus -35 region of the trp promoter. However, the four base pairs between these two base pairs in pKGP36 differ from those in the trp promoter. The distance (spacer) between the -35 and the -10 regions of pKGP36 is 17 base pairs, the same distance as in the trp promoter. This novel promoter then has the basic structure to function as a promoter, but with less efficiency than the trp promoter. This can be an advantage when the ultimate objective is to fuse foreign genes such as IFN-ß to an efficient promoter such as the trp or lac promoters.

Another preferred embodiment of this invention is the plasmid depicted in Figure 5, pAP16. Plasmid vector pAP16, molecular weight of about 4.8 x $10^6$ daltons, represents a general purpose vector useful for fusion of a foreign gene to P.O.-S.D. sequences from pKGP36. The ß-galactosidase gene employed does not have the initiating ATG codon, and it cannot direct expression of ß-galactosidase alone. The ß-galactosidase gene, from pLG400, was fused to P.O.-S.D. control elements from pKGP36 to form pAP16. The vector gives non-detectable levels of ß-galactosidase. However, when a functional amino terminal gene fragment is inserted in frame between the P.O.-S.D. control element and the ß-galactosidase gene, a fused protein can be produced which shows ß-galactosidase activity. This greatly facilitates the selection of foreign gene fusion to the P.O. of pKGP36.

Plasmid pAP16 is the result of a fusion between the PstI/HindIII P.O. containing fragments of pKGP36, and the PstI/HindIII ΔlacIZ-containing

18

fragment of plasmid pLG400. The following restriction map further characterizes this plasmid: PstI-HindIII, 1300 bp; ClaI-ClaI, 600 bp; HpaI-HindIII, 39 bp; and HpaI-BamHI, 45 bp. Plasmid pAP16 comprises the ΔlacIZ sequences fused to the promoter-operator sequences of plasmid pKGP36 to create a unique vector for the fusion of foreign genes to the P.O. and leader S.D. sequences and in frame with the ΔlacIZ genes. Expression from the promoter-operator yields a fused gene product, e.g., pAPFIF9 (see Figure 6), detectable by typical ß-galactosidase assays. The subsequent plasmid is then used to manipulate the sequences between the tryptophan leader S.D. and the foreign gene of interest. The plasmid can also be used to study the expression of the heterologous genes in E. coli. Another use of the plasmid is the fusion of antigenic determinants to the ΔlacIZ gene. This allows the synthesis of fused antigens for diagnostics or vaccines.

Plasmid pAP16 can be used for the cloning and manipulation of any foreign gene sequence carrying its own ATG initiation codon when fused in frame to the ΔlacIZ gene. Plasmid pAP16 offers an alternative to using the lac P.O. and a Δlac strain of E. coli. Because ß-galactosidase activity expression from pAP16 derivatives carrying a fusion protein such as pAPFIF9 is under the control of the tryptophan operator, plasmid directed ß-galactosidase activity can be measured above background in any E. coli host.

The lac P.O. fusion systems require specific strains containing deletions of the chromosomal ß-galactosidase gene. This is necessary because the same inducers (lactose and IPTG) induce expression of

both the plasmid ß-galactosidase and the chromosomal ß-galactosidase genes. Therefore, the plasmid activity was indistinguishable from chromosomal activity. With pAP16, expression of plasmid directed ß-galactosidase activity is inducible by depleting the medium of tryptophan or by the addition of ß-IAA. These induction conditions allow for the expression of plasmid directed ß-galactosidase activity without turning on chromosomal ß-galactosidase expression. ß-Galactosidase deletion strains such as LG-90 gave a very low efficiency of correctly assembled molecules (5 to 10%). Alternatively, strains containing the lacZ gene, MM294 and HB101, when transformed with the same ligation mix produced correctly assembled molecules with efficiencies of 90 and 95% respectively.

Another embodiment of this invention is plasmid pAPFIF9 depicted in Figure 6. This plasmid, having a molecular weight of about $5.1 \times 10^6$ daltons, has the restriction map: PstI-HpaI, 1320 bp; HpaI-HindIII, 39 bp; HindIII-PstI, 207 bp; PstI-HindIII, 270 bp; and HindIII-BamHI, 6 bp. In this plasmid, the IFN-ß amino terminus coding sequence is fused between the PKGP36 P.O.-S.D. control elements and the ß-galactosidase gene.

Restriction mapping, DNA sequence analysis, and biological assay methods were employed to show that a fused protein with ß-galactosidase activity is being directed by the IFN-ß initiation codon, ATG. Manipulation of the DNA between the P.O.-S.D. control elements and the IFN-ß ATG codon can be carried out to maximize the production of the fused protein, monitored by the ß-galactosidase assay. Once optimal expression of the fused protein has been obtained from the PKGP36 P.O.-S.D. sequences, reconstitution

20

of authentic IFN-ß gene can be obtained by replacing the ΔlacIZ sequences with the IFN-ß carboxyl terminal gene fragment (Figure 7). The tryptophan P.O. can be reconstituted from the pKGP36 P.O. for efficient expression of IFN-ß or other foreign genes. Other preferred embodiments include pKGP13-19R and pKGP13-19R•trp6.

The plasmids pKGP12-2, 13-19, and 9-25 are characterized by molecular weights of about 5.1 x $10^6$ daltons and the restriction map: PstI-HpaI, 1320 bp; HpaI-ClaI, 27 bp; ClaI-PstI, 145 bp; PstI-HindIII, 270 bp; and HindIII-BamHI, 6 bp.

The plasmids pKGP12-2R, 13-19R and 9-25R have molecular weights of about 3.4 x $10^6$ daltons, and the restriction map: PstI-HpaI, 1330 bp; HpaI-ClaI, 27 bp; and ClaI-PstI, 145 bp. Plasmid pKGP13-19R•trp6 has a molecular weight of about 3.7 x $10^6$ daltons and the restriction map: PstI-HpaI, 1530 bp; HpaI-ClaI, 27 bp; ClaI-PstI, 145 bp.

Plasmid pBREIL-2 is characterized as follows. It has a molecular weight of about 1.7 x $10^6$ daltons and the restriction map: HindIII-IL-2 StuI site including IL-2 gene, 450 bp; IL-2 StuI site-HindIII excluding IL-2 gene, 2325 bp.

Plasmid pKGP36•trp is characterized by a molecular weight of about 3.0 X $10^6$ daltons and the restriction map: HindIII-PstI, 3586 bp; PstI-ClaI, 775 bp; ClaI - ClaI including Trp P.O., 790 bp; Cla I-HindIII, 4 bp.

Plasmid pTrpEIL-2 is characterized by a molecular weight of about 2.1 x $10^6$ daltons and the restriction map: HindIII-Pst I, including IL-2 gene, 2000 bp; Pst I-HindIII including Trp P.O., 1569 bp.

Microorganisms suitable for transformation include bacteria of the Escherichia coli genus.

Examples of suitable strains include K-12, HB101, MM294, and RR1 with strain K-12 being preferred.

Methods

As will be appreciated by those skilled in the art, the editing, restructuring and other manipulations described herein are carried out between the control elements and the first ATG codon which initiates translation of the desired protein. These manipulations are carried out on the amino terminal (front portion) of the gene. If a gene coding for an easily assayable protein is fused to the amino terminal of a foreign gene the restructuring events of interest can be easily assayed. For our purposes, we select the ß-galactosidase gene and the tryptophan operon control elements from E. coli. The level of enzyme activity is directly proportional to the number of ß-galactosidase molecules present. Thus, by fusing the ß-galactosidase gene to the amino terminal of, for example, the IFN-ß gene, the levels of the hybrid molecule expressed in E. coli can be determined by a simple colorimetric assay.

The plasmids pKGP43 and pKGP36 were obtained by the following process. The E. coli tryptophan promoter-operator, Shine-Dalgarno DNA sequences were excised from plasmid pLD102 by digestion with restriction enzymes HpaII and BalI. A fragment containing the desired DNA was purified and modified with the restriction enzyme TaqI. The resulting modified fragments were subcloned into the ClaI restriction site of plasmid pBR322. The above construction gave rise to two expression plasmids containing sequences from the trp operon in opposite orientations. Plasmid pKGP43 can direct the expression of genes subcloned into the restriction sites EcoRI and PstI or any site within the ß-lactamase gene. Plasmid pKGP36 contains a novel hybrid tryptophan P.O. and can direct the expression in low levels of genes subcloned into the restriction sites HindIII, or any site within the tet$^R$ gene.

The novel plasmids of the invention can be employed for the overproduction of a variety of homologous and heterologous polypeptides when an appropriate gene is inserted into the plasmids. It is preferred to employ the plasmids for the overproduction of interleukin-2, ß-lactamase and human fibroblast interferon. The use of plasmid pKGP43 for the production of ß-lactamase, the use of plasmid pKGP13-19R•trp6 for the production of IFN-ß, and the use of plasmid pTrpEIL-2 for the production of interleukin-2 are particularly preferred. ß-Lactamase has utility as a screening agent for drug research and as an agent to clear blood samples of residual penicillins in preparation for analysis. Interferon is a natural molecule produced by cells in response to virus infection. This molecule, when exposed to cells, renders them resistant to virus infection. Interleukin-2 is a lymphokine which is capable of influencing cell-mediated immune responses in mammals. For example, it functions as a regulator of T-lymphocytes.

Plasmids of this invention have been deposited at the American Type culture collection, Maryland U.S.A. The dates of deposition and accession numbers are as follows:

| Plasmid | ATCC Accession Number | Date of Deposition |
|---|---|---|
| pKGP36 | 39413 | 10 August 1983 |
| pKGP43 | 39414 | " |
| pAP16 | 39415 | " |
| pKGP13-19R | 39416 | " |
| pKGP13-19R•trp6 | 39412 | " |
| pTrpEIL-2 | 39750 | 26 June 1984. |

23

The following are illustrative Examples of the invention in which all parts and percentages are by weight and all degrees are Celsius unless otherwise noted.

## EXAMPLE 1

Construction of pKGP43 and pKGP36. Plasmid pLD102 was amplified in a 1 liter culture of host E. Coli W3110 trp C55 and purified by a standard cleared lysate, CsCl-ethidium bromide equilibrium centrifugation procedure. Two µg of the plasmid was digested with restriction enzymes HpaII and BalI and separated on a 5% acrylamide gel. Several fragments migrating near the 400 base pair (bp) range were observed. One fragment disappeared after further digestion of the HpaII/BalI reaction with HpaI, indicating the presence of the trp P.O.-S.D. sequences in the HpaII/BalI fragment. This fragment was then purified and subjected to a limited digestion with TaqI to determine if the expected TaqI restriction sites were present. Polyacrylamide gel analysis of the TaqI digest indicated that the expected sites were present.

The 400 bp fragment containing the HpaI site (Figure 13) was partially digested with TaqI as follows: 250.0 ng fragment was digested with 1 unit of TaqI for 12 minutes at 65° in a final volume of 10 µl. Plasmid pBR322 was digested to completion with ClaI. Approximately 100 ng of vector DNA was combined with 4 µl of the partial TaqI digest of the 400 bp fragment in ligase buffer, 500 M of adenosine triphosphate (ATP), and 2 units of T4 DNA ligase in a final volume of 10 µl. The above mixture was incubated at 12° for 48 hrs.

24

## Transformation and Screening

E. Coli K-12 strains used for transformation were 294 (end A⁻, hsd R⁻, thi⁻, pro⁻) and HB101 (pro⁻leu⁻thi⁻, lac y⁻, hsdR, end A⁻, rec A⁻, rps L20, ara 14, galK2, xyl-5, mtl-1, supE44). Cells to be transformed were grown in LB medium to an OD 550. of 0.5 in 20 ml of culture. The cells were pelleted in a centrifuge (Sorvall SS-34) rotor for 3 min at 5000 rpm, resuspended in 10 ml of 50 mM $CaCl_2$ and incubated on ice for 1 hr. The cells were then repelleted and resuspended in 2 ml of 50 mM $CaCl_2$. Competent cells were then incubated on ice an additional 30 minutes. They were used directly for transformation, or stored overnight in the $CaCl_2$ solution and used 24 hrs later. Ten μl of the ligation mix described above was added to 200 μl of competent cells, and the mixture was incubated at 37° for 5 min. LB medium (0.8 ml) was added to the cell DNA mix which was then incubated at 37° for 1 hr. Aliquots (100μl) were spread on LB plates containing 25 μg/ml of ampicillin. Plasmid DNA was prepared by the method of Birnboim and Doly, Nucleic Acids Res. 7, pages 1513 to 1523 (1979). Plasmids containing the tryptophan promoter-operator and Shine-Dalgarno sequences were designated pKGP.

## Physical Characterization of Expression Vectors

Plasmid DNA was purified by standard methods (Clewell) and ranged in size from 4400 bp to 4950 bp. Plasmids with a single HpaI restriction site and inserts of 100 bp or greater were subjected to restriction enzyme analysis to determine the orientation of the tryptophan promoter fragment and its position in relation to unique restriction sites within pBR322. Restriction sites, distances between restriction sites and fragment orientation were

25

determined by agarose and polyacrylamide gel electrophoresis of restriction enzyme digestions. Vectors, pKGP43, 44, 45, and pKGP36 were chosen for further study.

Efficacy of Expression Vectors

Expression vector pKGP43 had the tryptophan P.O.-S.D. fragment oriented so that the ß-lactamase gene was under control. This was deduced from the restriction map of Figure 3. By analyzing the expression of ß-lactamase the efficacy of the expression vector was demonstrated. In the first experiment 3 clones, pKGP43, 44, and 45 (pKGP44 and 45 are independent isolates of the same construction as pKGP43) were grown with shaking in LB media to about $5 \times 10^8$ cells/ml. ß-Indoleacrylic acid was added to a final concentration of 20 µg/ml, and the cells were shaken at 37° for 2 hours. A control culture was grown under the same conditions in the absence of ß-IAA. One ml of culture was centrifuged, and the cells were resuspended in M9 media to which 0.5% of casamino acids and 25 µg/ml of ampicillin were added. Fifty µCi $^{35}$S methionine was added to the cultures and the cells were labeled for 30 minutes. After labeling, the cells were pelleted and resuspended in 50 µl of 100 mM tris(hydroxymethyl)aminomethane(tris) pH 6.8, 7.5% glycerol, and 1% sodium dodecyl sulfate (SDS).

The cell suspension was boiled for 3 minutes, pelleted, and samples were loaded on a 10% separating, 3% stacking polyacrylamide gel and electrophoresed at 25 ma constant current. After staining with Coomassie brilliant blue it was evident that a single band migrating in the range of about 29,000 daltons was overproduced in the culture incubated with ß-IAA when compared to the protein

0136090

26

profile of the same clone grown in the absence of
ß-IAA. The highest level of overproduction occurred
with clone pKGP44. The gel was then dried and
autoradiographed. In the culture of pKGP44 uninduced
with ß-IAA, the labeling pattern was similar to
pBR322. In the induced culture it was evident that a
disproportionate amount of labeling had occurred in
the 29,000 dalton protein band. ß-Lactamase enzyme
assays were performed before and after induction.
The enzyme activity of ß-lactamase directly
paralleled the induction of the 29,000 dalton
protein. These results are direct evidence that the
tryptophan P.O. contained within the construction is
capable of directing the overproduction of proteins
in E. coli.

Subsequent experiments were carried out on
E. coli which contained plasmids pKGP43, 44, and 45
to determine the best possible conditions for
overproduction of ß-lactamase. The pKGP45-containing
bacterium was grown overnight in M9 which contained
0.5% of casamino acids and 25 µg/ml of ampicillin.
The bacterium was induced for 4 hours in the presence
of ß-IAA. Protein analysis of the induced cultures
revealed a major band migrating at 29,000 daltons.
This band was not present in the pBR322 controls.
Densitometer tracings indicated that more than 80% of
new protein synthesis was ß-lactamase (putative
precursor and mature protein). Coomassie blue
staining of proteins from induced cells indicated
that more than 25% of total cellular protein was
ß-lactamase. DNA sequence analysis of pKGP43
revealed that the P.O. sequences matched the
published trp P.O. sequences as expected. See Figure
4A and Siebenlist et al., Cell 20, pages 269 to 281
(1980).

## EXAMPLE 2

Construction of pAP16. Plasmid purification, restriction enzyme digestions, ligations, transformations, and screening were carried out using the procedures described for these operations in Example 1.

Plasmid pLG400 was digested with PstI and HindIII. Plasmid pKGP36 was digested with PstI and HindIII and the promoter-operator-containing fragment was purified. The pKGP36 fragment was then ligated to the products of the pLG400 digest, and E. coli K-12, LG90, was transformed with the ligated DNA. DNA from selected transformants was prepared by the method of Birnboim and Doly loc cit, and subjected to restriction digestion with PstI/HindIII. The digests were electrophoretically separated on an 0.8% agarose gel and the correctly fused plasmids were identified. One of these, pAP16, was chosen for further study. Analysis confirmed that the HpaI/HindIII sites were separated by 39 bp and the HpaI/BamHI sites were separated by 45 bp as expected; see Figure 5.

## EXAMPLE 3

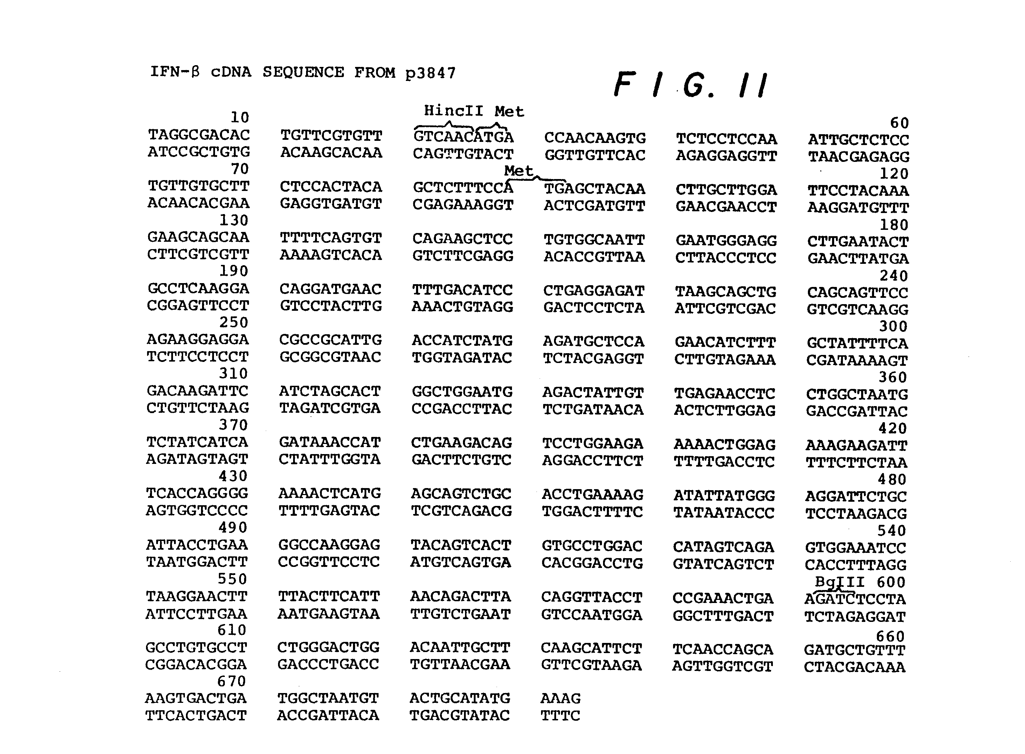

Construction of pAPFIF9. An interferon cDNA-containing plasmid, plasmid 3847, was constructed by standard methods. The cDNA was synthesized by the general method of Wickens et al., as described in J. Biol. Chem. 253, pages 2483 to 2495 (1978), especially at page 2484. The cDNA sequence is depicted in Figure 11. Cloning was carried out by the standard method described by Villa-Komaroff et al., Proc. Natl. Acad. Sci. USA 75, pages 3727 to 3731 (1978), especially at pages 3727 and 3728. Plasmid 3847 was digested with HincII and BglII and the IFN-ß cDNA fragment was purified after electrophoretic separation on a polyacrylamide gel. The HincII/BglII fragment (Figure 6;A) was subjected to further digestion by HaeIII which cut the IFN-ß cDNA at base 471 of the mature coding sequence (Figure 6;B). The HincII/HaeIII IFN-ß cDNA fragment was purified after electrophoretic separation on a polyacrylamide gel. HindIII linkers (CAAGCTTG) were ligated to both ends of the HincII/HaeIII IFN-ß cDNA fragment.

The ligation products (Figure 6;C) were digested with HindIII and the desired IFN-ß cDNA fragment now containing HindIII restriction sites on each end was purified from a polyacrylamide gel after electrophorectic separation. Plasmid pAP16 was digested with HindIII. The IFN-ß cDNA fragment was ligated into the vector, and E. coli K-12, LG-90, was transformed with the ligated DNA. Plasmid DNA was prepared from selected transformants as described for construction of pAP16, and it was digested with PstI. Since the vector had only one PstI site and the IFN-ß cDNA contained a single PstI site, plasmids with two PstI sites were selected for further analysis.

Restriction mapping revealed that the fragment was inserted in both possible orientations. One recombinant plasmid, pAPFIF9, contained the initiation codon of IFN-ß cDNA fused in the proper orientation to the P.O. fragment of pAP16. The IFN-ß cDNA was fused in frame to the ΔlacIZ gene, constructing a IFN-ß ß-galactosidase fusion gene. The opposite orientation was named pAPFIF8. Further analysis showed that pAPFIF9 produced ß-galactosidase activity while pAPFIF8 did not. Also pAPFIF9 coded for a fused protein as expected while pAPFIF8 did not.

EXAMPLE 4

Construction of pKGP12-2 and pKGP13-19.

Plasmids pAPFIF9 and pAP16 were used to clone, edit, and express IFN-ß in E. coli. In order to express active IFN-ß in E. coli, the DNA sequences coding for the IFN-ß signal sequence were removed in two ways. Using a modification of the method of Goeddel et al., Nucleic Acid Res. 8, pages 4057 to 4074 (1980), a specific oligonucleotide ATGAGCTACAAC was hybridized to one strand of a denatured HincII/BglII fragment of IFN-ß cDNA. Utilizing the polymerizing activity of the Klenow fragment of DNA polymerase a new strand was synthesized with the oligonucleotide as primer, through the IFN-ß PstI site. Subsequently in a separate reaction, 3' $\longrightarrow$ 5' exonuclease activity degraded the single stranded signal region stopping at the duplex formed by the oligonucleotide and the IFN-ß cDNA. This modification of the method of Goeddel et al. was necessary to obtain sufficient yields of the edited fragment for cloning. After digestion of the reaction products and separation on a polyacrylamide gel, the expected 140 bp blunt end - PstI band was purified and assembled into an expression vector as follows.

Plasmid pAP16 was first digested with HindIII and the HindIII sites filled in to yield a blunt end. This reaction was followed by digestion with PstI. The blunt end PstI fragment containing the amino terminus of the ß-lactamase gene and the pKGP36 P.O.-S.D. fragment was purified from a polyacrylamide gel. Plasmid pAPFIF9 was digested with PstI and the PstI fragment containing the carboxyl terminal of the ß-lactamase gene and the carboxyl terminal of IFN-ß cDNA fused to ΔlacIZ gene was purified. The pAP16 PstI blunt end

30

fragment, the pAPFIF9 PstI fragment and the edited PstI-blunt end IFN-ß fragment were assembled via a three-way ligation as shown in Figure 7.

E. coli K-12, LG90, was transformed with the ligated molecules. Properly assembled molecules appeared blue on M9 Xgal plates because of the contribution from the IFN-ß/ΔlacIZ fusion obtained from pAPFIF9. The proper constructions were confirmed by restriction mapping and DNA sequence analysis. Two clones pKGP12-2 and pKGP13-19 were identified in this manner. The PstI fragment from pAPFIF9 provided the desired selection for the proper fusion of IFN-ß edited cDNA to the pKGP36 promoter. All blue colonies contained properly constructed molecules demonstrating the utility of pAP16 plasmid.

EXAMPLE 5

Construction of pKGP9-25. An alternative editing method to that described in Example 4 was employed using pAPFIF9 and Bal 31 exonuclease. Plasmid pAPFIF9 was digested with HpaI restriction enzyme. This gave three fragments, one of which contained the IFN-ß portion of the fused gene. These fragments were randomly degraded with Bal 31 exonuclease by standard procedures and digested with BamHI. This gave a set of fragments randomly degraded at the 5' end of the IFN-ß gene and ending in a unique BamHI site. Only IFN-ß fragments deleted at the 5' end had an in frame ATG to initiate translation and ended in a BamHI site that would fuse the IFN-ß fragment in frame with the ß-galactosidase gene of pAP16. The vector pAP16 was digested with HindIII, and staggered ends were filled in with the Klenow fragment of DNA polymerase in the presence of deoxynucleotide triphosphates to yield blunt ends. The blunt-ended molecules were then digested with BamHI. The randomly degraded IFN-ß fragments were ligated into the modified pAP16 vector, and the DNA was used to transform E. coli K-12, LG90 and E. coli HB101 as outlined in Figure 8.

Resultant clones were screened as described for pKGP12-2 and pKGP13-19. Clone pKGP9-25 contained a plasmid with the pre IFN-ß sequences deleted and the ATG initiating the mature active protein fused to the promotor-operator fragment of pAP16 as expected. All of the constructions were confirmed by DNA sequence analysis.

## EXAMPLE 6

### Construction of IFN-ß expressing plasmids pKGP12-2R, 13-19R and 9-25R from fusion plasmids pKGP12-2, 13-19, and 9-25.

In order to obtain IFN-ß expressing plasmids the carboxyl terminal of the edited IFN-ß gene from plasmids pKGP13-19, 12-2, and 9-25 that is fused to the ΔlacIZ gene must be replaced with the functional carboxyl terminal sequence of the IFN-ß gene. All three of the subject fusion plasmids were converted to IFN-ß expression plasmids by the same method. Only the conversion of pKGP13-19 will be described hereafter.

The PstI/BamHl fragment from pBR322 was purified. This fragment contained the carboxyl terminal of ß-lactamase and the origin of replication of pBR322. The PstI fragment from pKGP13-19 containing the amino terminal of ß-lactamase and the P.O. of pKGP36 fused to the edited amino terminal of IFN-ß was purified. Finally, the PstI/BglII carboxyl terminal IFN-ß fragment was purified. These three fragments were assembled in a three-way ligation (Figure 9), and the DNA was used to transform E. coli MM294. Plasmid DNA was prepared and digested with PstI followed by analysis on agarose gels. Clones containing plasmids with two PstI sites separated by about 1000 base pairs were sequenced and assayed for IFN-ß activity. DNA sequencing was carried out by cloning selected fragments into M13 phage DNA and DNA sequence was determined by the dideoxy method using reagents supplied by New England Biolabs.

In order to assay for IFN-ß activity, bacteria were grown to an O.D. of about 1 and induced by shaking for about 2 hrs in the presence of 20 µg/ml of ß-IAA. Cells were concentrated 100-fold and lyzed by sonication. Cell debris was pelleted and the supernatant was assayed for antiviral activity using the inhibition of cytopathic effect assay described by Knight et al., J. Interferon Res. 2, pages 421 to 429 (1982). Plasmids pKGP12-2R, 13-19R and 9-25R had the expected DNA sequence (Figure 10) and expressed antiviral activity. The antiviral activity was resistant to pH2, sensitive to heating at 56° and neutralized by specific antisera to IFN-ß. Thus, this Example demonstrates the cloning and expression of IFN-ß in E. coli using the described cloning and expression vectors.

## EXAMPLE 7
### Construction of pKGP13-19R•trp6.

Transcription of the IFN-ß gene on plasmids pKGP12-2R, 9-25R and 13-19R is directed by the inefficient hybrid promoter from the plasmid pKGP36. In order to effect efficient transcription of the IFN-ß gene, the tryptophan promoter was reconstituted in a one-step cloning experiment. Plasmid pKGP43 contains the intact trp P.O. as described in Example 1. Restriction endonuclease HpaI cuts within the -10 regions of the trp and pKGP36 promoters. Plasmid pKGP43 was first digested with the restriction endonuclease HindIII. The HindIII

staggered ends were filled in as described in Example 4 to yield a fragment with two blunt ends. This fragment was then digested with HpaI, and the HpaI/filled in HindIII fragment containing the -35 region of the trp promoter was isolated. Plasmid pKGP13-19R was digested with HpaI. The HpaI/filled in HindIII fragment from pKGP43 containing the -35 region of the trp promoter was ligated into the HpaI site of pKGP13-19R, and the DNA was used to transform E. coli HB101.

Mini lysates were prepared from selected clones and the orientation of the inserted fragment was determined. DNA from clones with the correct orientation was sequenced. Sequence analysis confirmed that one clone, pKGP13-19R•trp6, contained the reconstituted trp promoter fused to IFN-ß gene as desired (Figure 10). Inhibition of cytopathic effect assays were carried out on pKGP13-19R and pKGP13-19R•trp6 by the method described by Knight et al., vide supra. Plasmid pKGP13-19R cultures contained about $5 \times 10^4$ u/liter of antiviral activity. Cultures of pKGP13-19R•trp6 contained about $10^7$ u/liter of antiviral activity. This clearly demonstrates the utility of the pKGP36 promoter for gene cloning and expression experiments. This system should prove useful for the cloning and manipulation of the foreign gene sequences that when expressed cause instability of the recombinant plasmid in culture.

## EXAMPLE 8

### Construction of pTrpEIL-2

An IL-2 cDNA containing plasmid, plH40, was constructed by standard methods as described for the interferon cDNA-containing plasmid, plasmid 3847, of Example 3.

34

In order to express active mature IL-2 at high levels in E. Coli, the IL-2 cDNA must be edited to remove the 5' DNA coding region for the signal sequence and the edited IL-2 cDNA must be inserted into an expression vector. Plasmid plH40 was digested with PstI, and the cDNA insert was purified. An HgiAI restriction site resides at the junction between the last amino acid of the signal sequence(SER) and the first amino acid of mature IL-2(ALA). The PstI fragment containing the IL-2 cDNA was cleaved with HgiAI. Since HgiAI leaves a four bp 3' overhang, the Klenow fragment of DNA polymerase was used to remove the four bases and leave a blunt end. This manipulation removed the ALA codon and left a blunt end beginning with CCT, the codon for the second amino acid (PRO) of mature IL-2.

In order to supply an initiating codon(ATG) as well as the codon for ALA, a specific adapter molecule, (A), was devised and synthesized. The fragment,

                    METALA
              CGATAAGCTTATGGCT
(A),    TATTCGAATACCGA, has several valuable features; it provides an AUG initiation codon as well as the first codon of mature IL-2. The CG overhang reconstituting a ClaI site and a HindIII site allowed insertion into the vector as follows. The adapter molecule was ligated to the blunt ended/PstI cDNA fragment described above, and the ligated DNA was digested with HindIII and StuI. The HindIII/StuI 450 bp fragment was purified on a polyacrylamide gel, and the purified fragment was ligated to the 2325 bp HindIII/PvuII fragment of pBR322. This construction was possible because restriction with StuI and PvuII left blunt ends. The DNA was then used to transform E. Coli MM294. Plasmid DNA was purified from selected clones and subjected to restriction analysis with XbaI (a single XbaI site resides within the IL-2 cDNA sequence). One vector, pBREIL-2, contained an XbaI site at the expected location and was chosen for further construction. The resulting vector, pBREIL-2, has increased stability because the IL-2 gene lacks an efficient promoter. The above constructions are outlined in Figure 14.

A derivative of vector pKGP36 (Example 1), pKGP36•trp, was constructed to facilitate the fusion of the IL-2 gene to the trp promoter, as follows: pKGP36 was digested with HpaI/PstI and the large fragment (3640 bp) containing the tet$^R$ gene, the origin of replication, and the carboxyl terminal of the ß-lactamase gene, was purified. Vector pKGP13-19R•trp 6 (Example 7) was digested with HpaI/PstI and the small fragment (1530 bp) containing the amino terminal of the ß-lactamase gene was purified. These two fragments were ligated and the DNA was used to transform E. Coli, MM294. Clones containing plasmids with a single HpaI site were chosen for further study. Plasmid DNA was purified from one clone and the DNA in the region of the trp promoter was sequenced confirming the fusion of the intact trp promoter. This vector was named pKGP36trp; 5160 bp (about 3.0 X 10$^6$ daltons).

The trp promoter was fused to the edited IL-2 gene using plasmids pKGP36•trp and pBREIL-2, as follows. The vector, pBREIL-2, was digested with HindIII/PstI and the large (2000 bp) fragment containing the IL-2 gene was purified. Plasmid pKGP36•trp was digested with HindIII/PstI and the small (1530 bp) fragment containing the trp promoter was purified. The two fragments were ligated and the DNA was used to transform E. Coli, MM294. Clones containing plasmids of the expected size with single HpaI and XbaI restriction sites were chosen for further analysis. DNA sequence analysis of one clone confirmed that the expected fusion of the trp promoter and the IL-2 gene had occurred; pTrpEIL-2, Figure 15. Total protein from E. Coli with and without the IL-2 expression vector was separated by polyacrylamide gel electrophoresis. A novel protein migrating with an apparent molecular weight of 15K daltons was present in cells containing the expression vector but was absent in control cells. The protein migrated as expected for unglycosylated IL-2. Lysates of cells containing pTrpEIL-2 were positive in radioimmunoassays using antibody to natural IL-2, and lysates of control cells were negative. IL-2 activity was determined by the IL-2 concentration dependent stimulation of proliferation (measured by $^3$H-thymidine incorporation) of a cloned murine cytotoxic T-lymphocyte line (CTLL-2, subclone 15H). Extracts of pTrpEIL-2-containing cells gave between 3000 and 5000 relative reference units per ml of culture while control extracts containing pKGP36•trp gave no activity. Antibody to natural IL-2 neutralized the activity in the recombinant extracts. This proved that the IL-2 gene inserted into the expression vector directed the synthesis of high levels of recombinant protein with the properties of human IL-2.

CLAIMS:

1. A promoter-operator Shine-Dalgarno ClaI-containing fragment comprising about 600 base pairs and having a -10 region and a Shine-Dalgarno sequence characteristic of the trp promoter-operator Shine-Dalgarno fragment, and comprising the following sequences:

(i) a 6-base pair sequence in the -35 region of the fragment (5'——→ 3') of

$$\begin{array}{cccccc} T & G & C & C & G & A \\ A & C & G & G & C & T \end{array}$$ , and

(ii) a sequence of about 17 base pairs between the -35 and the -10 regions of said fragment, the sequence of the first eight base pairs (5'——→ 3') being

$$\begin{array}{cccccccc} C & G & C & G & C & A & T & C \\ G & C & G & C & G & T & A & G \end{array}$$ .

2. A plasmid comprising a fragment according to Claim 1.

3. A plasmid according to Claim 2 comprising, additionally, one of: (i) a gene that codes for ß-lactamase, or (ii) genes that code for IFN-ß and ß-lactamase.

4. A plasmid according to Claim 3, pKGP36, comprising a gene that codes for ß-lactamase and a gene that codes for tetracycline resistance, the promoter-operator being oriented toward the tet$^R$ gene, the molecular weight of the plasmid being about $3.0 \times 10^6$ daltons, the plasmid having the following partial restriction map: PstI-EcoRI, 750bp; EcoRI-ClaI, 23bp; ClaI-HpaI, 565bp; ClaI-ClaI, 600bp; HpaI-ClaI, 35bp; HpaI-HindIII, 39bp; HindIII-BamHI, 345 bp.

5. A plasmid according to Claim 2, pAP16, comprising a ß-galactosidase gene lacking an initiating ATG codon, the promoter-operator being oriented toward said gene, the molecular weight of the plasmid being about 4.8 x $10^6$ daltons, the plasmid having the following restriction map: PstI-HindIII, 1300bp; ClaI-ClaI, 600bp; HpaI-HindIII, 39bp; HpaI-BamHI, 45bp.

6. A plasmid according to claim 2, pAPFIF9, comprising a fused gene that codes for $\beta$-galactosidase and the amino terminus of IFN-$\beta$, the promoter-operator being oriented toward the IFN-$\beta$ amino terminus coding sequence, the molecular weight of the plasmid being about 5.1x$10^6$ daltons, the plasmid having the following restriction map: PstI-HpaI, 1320bp; HpaI-HindIII, 39bp; HindIII-PstI, 207bp; PstI-HindIII, 270bp; and HindIII-BamHI, 6bp.

7. A plasmid according to Claim 2, selected from the group pKGP12-2, pKGP13-19, and pKGP9-25, comprising a fused gene that codes for ß-galactosidase and the amino terminus of IFN-ß, the gene coding for IFN-ß having been edited to remove its signal sequence, the promoter-operator being oriented toward the edited IFN-ß gene, the molecular weight of the plasmid being about 5.1 x $10^6$ daltons, the plasmid having the following restriction map: PstI-HpaI, 1320 bp; HpaI-ClaI, 27 bp; ClaI-PstI, 145 bp; PstI-HindIII, 270 bp; HindIII-BamHI, 6bp.

8. A plasmid according to Claim 2, selected from the group pKGP12-2R, pKGP13-19R, and pKGP9-25R, comprising a gene that codes for biologically active IFN-ß, the promoter-operator being oriented toward the IFN-ß gene, the molecular weight of the plasmid being about 3.4 x $10^6$ daltons, the plasmid having the following restriction map: PstI-HpaI, 1330bp; HpaI-ClaI, 27bp; ClaI-PstI, 145bp.

9. A plasmid, pKGP43, comprising a gene that codes for ß-lactamase and a gene that codes for tetracycline resistance, comprising also a tryptophan promoter-operator Shine-Dalgarno sequence oriented toward the ß-lactamase gene, the molecular weight of the plasmid being about $2.8 \times 10^6$ daltons, the plasmid having the following partial restriction map: PstI-EcoRI, 750bp; EcoRI-HpaI, 100bp; HpaI-HindIII, 300 bp; HindIII-BamHI, 345bp.

10. A plasmid, pKGP13-19R•trp6, comprising a gene that codes for biologically active IFN-ß, the plasmid having a tryptophan promoter-operator Shine-Dalgarno sequence oriented toward the IFN-ß gene, the molecular weight of the plasmid being about $3.7 \times 10^6$ daltons, the plasmid having the following restriction map: PstI-HpaI, 1530bp; HpaI-ClaI, 27bp; ClaI-PstI, 145bp.

11. A plasmid, pBREIL-2, comprising a gene that codes for ß-lactamase and a gene that codes for IL-2, the molecular weight of the plasmid being about $1.7 \times 10^6$ daltons, the plasmid having the following partial restriction map: HindIII-IL-2 StuI site including IL-2 gene, 450 bp; IL-2 StuI site-HindIII excluding IL-2 gene, 2325 bp.

12. A plasmid, pKGP36•trp, comprising a gene that codes for ß-lactamase and a gene that codes for tetracycline resistance, the trp promoter-operator being oriented toward the tet$^R$ gene, the molecular weight of the plasmid being about $3.0 \times 10^6$ daltons, the plasmid having the partial restriction map: HindIII-PstI, 3586 bp; PstI-ClaI, 775 bp; ClaI - ClaI including Trp P.O., 790 bp; Cla I-HindIII, 4 bp.

0136090

40

13. A plasmid, pTrpEIL-2, comprising a gene that codes for β-lactamase and a gene that codes for IL-2, the molecular weight of the plasmid being about $2.1 \times 10^6$ daltons, the plasmid having the following partial restriction map: HindIII-Pst I including IL-2 gene, 2000 bp; Pst I-HindIII including Trp P.O., 1569 bp.

14. A method for making a plasmid selected from pKGP43 and pKGP36 as claimed in claims 4 and 9, comprising the steps of

    (i)    restricting a plasmid of the pLD102 type using HpaII and BalI;

    (ii)   isolating and purifying the trp-promoter-operator-containing HpaII/BalI fragment;

    (iii) modifying the HpaII/BalI fragment by limited digestion with TaqI; and

    (iv)   inserting said modified fragment into the ClaI site of pBR322.

15. A method for making plasmid pAP16, as claimed in claim 5, comprising the steps of:

    (i)    restricting a plasmid of the pLG400 type using PstI and HindIII to obtain a PstI/HindIII fragment;

    (ii)   restricting a plasmid of the pKGP36 type with PstI and HindIII, and isolating and purifying the promoter/operator-containing fragment; and

    (iii) ligating the promoter-operator containing fragment of pKGP36 to the PstI/HindIII fragment of pLG400.

16. A method for making plasmid pAPF1F9, as claimed in claim 6, comprising the steps of:

(i)     restricting the IFN-ß cDNA fragment with
        HaeIII/HincII to obtain a HaeIII/HincII
        fragment, and isolating and purifying said
        fragment;

(ii)    ligating HindIII linkers to both ends of the
        HaeIII/HincII fragment of step (i) to obtain
        a HindIII/HindIII fragment, and isolating
        and purifying said fragment;

(iii)   restricting a plasmid of the pAP16 type
        using HindIII; and

(iv)    ligating the fragment of step (ii) with the
        fragment of step (iii).

17.     A method for making a plasmid selected from
pKGP12-2 and pKGP 13-19, as claimed in claim 7,
comprising the steps of:

(i)     restricting a plasmid of the pAP16 type
        using HindIII, and filling in the HindIII
        site with the Klenow fragment of E. coli DNA
        polymerase to obtain a blunt-ended fragment;

(ii)    restricting the fragment of step (i) using
        PstI to obtain a PstI fragment containing
        the amino terminal of the ß-lactamase (AMP)
        gene and the pKGP36 tryptophan
        promoter-operator Shine-Dalgarno (P.O.-S.D.)
        unit, and purifying said fragment;

(iii)   restricting a plasmid of the pAPF1F9 type
        using PstI, and purifying the fragment which
        contains the carboxyl terminal of the
        ß-lactamase gene and the carboxyl terminal
        of IFN-ß cDNA;

(iv)    editing the IFN-ß cDNA fragment giving a 140
        base pair blunt ended ATG/PstI fragment; and

(v)     ligating the fragments of steps (ii), (iii)
        and (iv).

18. A method for making plasmid pKGP9-25, as claimed in claim 7, comprising the steps of:

(i) restricting a plasmid of the pAP16 type using HindIII, and filling in the HindIII site with the Klenow fragment of E. coli DNA polymerase to obtain a blunt-ended fragment;

(ii) digesting the fragment of step (i) with BamHI;

(iii) restricting a plasmid of the pAPFIF9 type with HpaI to give three fragments;

(iv) treating the fragments of step (iii) with Bal 31 exonuclease and BamHI to give fragments randomly degraded at the 5' end of the IFN-ß gene and ending in an BamHI site;

(v) ligating the IFN-ß fragments of step (iv) into the pAP16 fragment of step (ii).

19. A method for making plasmids pKGP12-2R, pKGP13-19R, and pKGP9-25R, as claimed in claim 7, comprising the steps of:

(i) restricting a plasmid of the pBR322 type using PstI and BamHI to obtain a PstI/BamHI fragment and purifying said fragment;

(ii) restricting a plasmid of the (a) pKGP12-2, (b) pKGP13-19, or (c) pKGP9-25 type using PstI, and purifying the fragment which contains the amino terminal of ß-lactamase the pKGP36 P.O.-S.D. unit and the edited amino terminal of the IFN-ß gene;

(iii) restricting a plasmid of the p3847 type using PstI and BglII and purifying the fragment which contains the carboxyl terminal fragment of the IFN-ß gene; and

(iv) ligating the fragments of steps (i), (iii), and (a) (b) or (c) of step (ii).

20. A method for making plasmid pKGP13-19R•trp6, as claimed in claim 10, comprising the steps of:

(i)  restricting a plasmid of the pKGP43 type using HindIII and filling in the HindIII sites with the Klenow fragment of E. coli DNA polymerase to obtain a blunt-ended fragment;

(ii)  restricting the fragment of step (i) using HpaI and isolating the HpaI/ filled in HindIII fragment containing the -35 region of the trp promoter;

(iii) restricting a plasmid of the pKGP13-19R type using HpaI; and

(iv)  ligating the fragment of step (ii) with the fragment of step (iii)

21. A method for making plasmid pBPEIL-2, as claimed in claim 11, comprising the steps of:

(i)  restricting a plasmid of the pIH40 type with PstI to produce a cDNA fragment containing the gene for IL-2;

(ii)  treating the fragment of step (i) to form a blunt 3' end beginning with the CCT codon;

(iii) ligating to the blunt 3' end of the fragment of step (ii) an adapter molecule comprising ATG and GCT codons in serial arrangement and containing a HindIII/site;

(iv)  restricting the fragment of step (iii) with HindIII and StuI;

(v)  restricting a plasmid of the pBR322 type with HindIII and PvuII, and isolating the large DNA fragment;

(vi)  ligating the IL-2 gene-containing fragment of step (iv) to the HindIII/PvuII fragment of step (v).

22.  A method for making plasmid pKGP36-trp, as claimed in claim 12, comprising the steps of:

   **(i)   restricting a plasmid of the pKGP36 type with HpaI and PstI and purifying the fragment containing the tet$^R$ gene;**

   **(ii)  restricting a plasmid of the pKGP13-19R•trp-6 type with HpaI and PstI, and purifying the fragment containing the trp P.O.;**

   **(iii) ligating the fragments of steps (i) and (ii).**

23.  A method for making    plasmid pTrpEIL-2 as claimed in claim 13, comprising the steps of:

   **(i)   restricting a plasmid of the pBREIL-2 type with HindIII and PstI, and purifying the fragment containing the IL-2 gene;**

   **(ii)  restricting a plasmid of the pKGP36•trp type with HindIII and PstI, and purifying the fragment containing the trp P.O.;**

   **(iii) ligating the fragments of steps (i) and (ii).**

24.  A mutated plasmid derived from a plasmid as claimed in any of claims 2 to 13 which is nevertheless a functional expression plasmid.

25.  A method for modifying bacterial microorganisms which comprises inserting a plasmid as claimed in any of claims 2 to 13 and claim 24.

26.  A method as claimed in claim 25 which comprises the steps of:

   **(i)   growing bacterial cells to a designated optical density;**

   **(ii)  modifying the cell walls by treating with calcium chloride solution;**

0136090

45

(iii) mixing competent cells with ligated DNA
mixture containing the plasmid; and

(iv) incubating the mixture

27. A microorganism transformed by a plasmid as claimed in any of claims 2 to 13 and claim 24.

28. An E. coli bacterium transformed by a plasmid as claimed in any of claims 2 to 13 and claim 24.

29. A method for over-producing proteins from a transformed microorganism as claimed in claim 27, which comprises the steps of:

(i) growing the transformed microorganism in an enriched medium to an optical density of at least 2 at 600 nm;

(ii) diluting the grown cells 1:10 in minimal media; and

(iii) treating the diluted cells with $\beta$-indoleacrylic acid.

30. Molecule (A) comprising an initiation codon, the first codon of mature IL-2, and having the sequence:

CGATAAGCTTATGGCT
TATTCGAATACCGA.

0136090

1 / 12

# F I G. 1 (Prior Art)

pLD102
5.8 X $10^6$d

pACYC177 VECTOR

# F I G. 13

# F I G. 2 (Prior Art)

pBR322

β-LACTAMASE

TrpPO EXPRESSION VECTOR ORIENTATION
TOWARD β-LACTAMASE GENE

**F I G. 3**

PO EXPRESSION VECTOR ORIENTATION
TOWARD tet$^R$ GENE

**F I G. 4A**

# FIG. 4B

pKGP36 PROMOTER-OPERATOR FRAGMENT

```
                              -35          SPACER        -10
TGGTCGGTGATCGCCAGGGTGCCGACGCGCATCTCGAACTAGTTAACTAGTACGCAAGTTCACGTAAAAAGGGTATCGA
      ***             **      *          ****************************************************
AATATTCTGAAATGAGCTGTTGACAATTAATCATCGAACTAGTTAACTAGTACGCAAGTTCACGTAAAAAGGGTATCG
                      -35                              HpaI
```

Trp PROMOTER-OPERATOR FRAGMENT

3 / 13

0136090

*F I G. 5*

## F I G. 6

# F I G. 7

# F I G. 8

# F I G. 9

## FIG. 10

# F I G. II

|  | | HincII Met | | | |
|---|---|---|---|---|---|
| TAGGCGACAC | TGTTCGTGTT | GTCAACATGA | CCAACAAGTG | TCTCCTCCAA | ATTGCTCTCC |
| ATCCGCTGTG | ACAAGCACAA | CAGTTGTACT | GGTTGTTCAC | AGAGGAGGTT | TAACGAGAGG |
| | | Met | | | |
| TGTTGTGCTT | CTCCACTACA | GCTCTTTCCA | TGAGCTACAA | CTTGCTTGGA | TTCCTACAAA |
| ACAACACGAA | GAGGTGATGT | CGAGAAAGGT | ACTCGATGTT | GAACGAACCT | AAGGATGTTT |
| GAAGCAGCAA | TTTTCAGTGT | CAGAAGCTCC | TGTGGCAATT | GAATGGGAGG | CTTGAATACT |
| CTTCGTCGTT | AAAAGTCACA | GTCTTCGAGG | ACACCGTTAA | CTTACCCTCC | GAACTTATGA |
| GCCTCAAGGA | CAGGATGAAC | TTTGACATCC | CTGAGGAGAT | TAAGCAGCTG | CAGCAGTTCC |
| CGGAGTTCCT | GTCCTACTTG | AAACTGTAGG | GACTCCTCTA | ATTCGTCGAC | GTCGTCAAGG |
| AGAAGGAGGA | CGCCGCATTG | ACCATCTATG | AGATGCTCCA | GAACATCTTT | GCTATTTTCA |
| TCTTCCTCCT | GCGGCGTAAC | TGGTAGATAC | TCTACGAGGT | CTTGTAGAAA | CGATAAAAGT |
| GACAAGATTC | ATCTAGCACT | GGCTGGAATG | AGACTATTGT | TGAGAACCTC | CTGGCTAATG |
| CTGTTCTAAG | TAGATCGTGA | CCGACCTTAC | TCTGATAACA | ACTCTTGGAG | GACCGATTAC |
| TCTATCATCA | GATAAACCAT | CTGAAGACAG | TCCTGGAAGA | AAAACTGGAG | AAAGAAGATT |
| AGATAGTAGT | CTATTTGGTA | GACTTCTGTC | AGGACCTTCT | TTTTGACCTC | TTTCTTCTAA |
| TCACCAGGGG | AAAACTCATG | AGCAGTCTGC | ACCTGAAAAG | ATATTATGGG | AGGATTCTGC |
| AGTGGTCCCC | TTTTGAGTAC | TCGTCAGACG | TGGACTTTTC | TATAATACCC | TCCTAAGACG |
| ATTACCTGAA | GGCCAAGGAG | TACAGTCACT | GTGCCTGGAC | CATAGTCAGA | GTGGAAATCC |
| TAATGGACTT | CCGGTTCCTC | ATGTCAGTGA | CACGGACCTG | GTATCAGTCT | CACCTTTAGG |
| | | | | | BgIII |
| TAAGGAACTT | TTACTTCATT | AACAGACTTA | CAGGTTACCT | CCGAAACTGA | AGATCTCCTA |
| ATTCCTTGAA | AATGAAGTAA | TTGTCTGAAT | GTCCAATGGA | GGCTTTGACT | TCTAGAGGAT |
| GCCTGTGCCT | CTGGGACTGG | ACAATTGCTT | CAAGCATTCT | TCAACCAGCA | GATGCTGTTT |
| CGGACACGGA | GACCCTGACC | TGTTAACGAA | GTTCGTAAGA | AGTTGGTCGT | CTACGACAAA |
| AAGTGACTGA | TGGCTAATGT | ACTGCATATG | AAAG | | |
| TTCACTGACT | ACCGATTACA | TGACGTATAC | TTTC | | |

10 / 13

0136090

# F I G. 12

DNA SEQUENCE OF THE TRANSCRIPTIONAL UNIT OF pKGP13-19R

```
                                                 HpaI
      10                                        ⌒⌒                    60
TGGTCGGTGA   TCGCCAGGGT   GCCGACGCGC   ATCTCGAACT   AGTTAACTAG   TACGCAAGTT
ACCAGCCACT   AGCGGTCCCA   CGGCTGCGCG   TAGAGCTTGA   TCAATTGATC   ATGCGTTCAA
      70  -S.D.-                      Met                           120
CACGTAAAAA   GGGTATCGAT   AAGCTATGAG   CTACAACTTG   CTTGGATTCC   TACAAAGAAG
GTGCATTTTT   CCCATAGCTA   TTCGATACTC   GATGTTGAAC   GAACCTAAGG   ATGTTTCTTC
      130                                                          180
CAGCAATTTT   CAGTGTCAGA   AGCTCCTGTG   GCAATTGAAT   GGGAGGCTTG   AATACTGCCT
GTCGTTAAAA   GTCACAGTCT   TCGAGGACAC   CGTTAACTTA   CCCTCCGAAC   TTATGACGGA
      190                                         PstI            240
CAAGGACAGG   ATGAACTTTG   ACATCCCTGA   GGAGATTAAG   CAGCTGCAGC   AGTTCCAGAA
GTTCCTGTCC   TACTTGAAAC   TGTAGGGACT   CCTCTAATTC   GTCGACGTCG   TCAAGGTCTT
      250                                                          300
GGAGGACGCC   GCATTGACCA   TCTATGAGAT   GCTCCAGAAC   ATCTTTGCTA   TTTTCAGACA
CCTCCTGCGG   CGTAACTGGT   AGATACTCTA   CGAGGTCTTG   TAGAAACGAT   AAAAGTCTGT
      310                                                          360
AGATTCATCT   AGCACTGGCT   GGAATGAGAC   TATTGTTGAG   AACCTCCTGG   CTAATGTCTA
TCTAAGTAGA   TCGTGACCGA   CCTTACTCTG   ATAACAACTC   TTGGAGGACC   GATTACAGAT
      370                                                          420
TCATCAGATA   AACCATCTGA   AGACAGTCCT   GGAAGAAAAA   CTGGAGAAAG   AAGATTTCAC
AGTAGTCTAT   TTGGTAGACT   TCTGTCAGGA   CCTTCTTTTT   GACCTCTTTC   TTCTAAAGTG
      430                                                          480
CAGGGGAAAA   CTCATGAGCA   GTCTGCACCT   GAAAAGATAT   TATGGGAGGA   TTCTGCATTA
GTCCCCTTTT   GAGTACTCGT   CAGACGTGGA   CTTTTCTATA   ATACCCTCCT   AAGACGTAAT
      490                                                          540
CCTGAAGGCC   AAGGAGTACA   GTCACTGTGC   CTGGACCATA   GTCAGAGTGG   AAATCCTAAG
GGACTTCCGG   TTCCTCATGT   CAGTGACACG   GACCTGGTAT   CAGTCTCACC   TTTAGGATTC
      550                                          Stop
GAACTTTTAC   TTCATTAACA   GACTTACAGG   TTACCTCCGA   AACTGA       
CTTGAAAATG   AAGTAATTGT   CTGAATGTCC   AATGGAGGCT   TTGACT
```

11/13

0136090

# F I G. 14

plH40

PstI    AsnSerAlaProThr           StuI          PstI

⌐ AACAGTGCACCTACT ───────────────┬─────────────┐
└ TTGTCACGTGGATGA ───────────────┴─────────────┘

Hgi Al          Hgi Al

ProThr                    StuI          PstI

CCTACT ───────────────────┬─────────────┐
ACGTGGATGA ───────────────┴─────────────┘

Klenow
3'→5'EXO

MetAla      ProThr        StuI      PstI

(A) CGATAAGCTTATGGCT + CCTACT ────────┬────────┐
     TATTCGAATACCGA     GGATGA ────────┴────────┘
     HindIII
oligonucleotide Adapter

Ligate
HindIII
StuI

MetAlaProThr              StuI
AGCTTATGGCTCCTACT ─────────────┐
    ATACCGAGGATGA ─────────────┘

HindIII
ClaI

PstI

AMP

pBR322

PvuII

HindIII

AMP        IL-2

PstI     pBREIL-2

# F I G. I5